# EUROPEAN PATENT SPECIFICATION

(11) **EP 1 687 314 B1**
(45) Date of publication and mention of the grant of the patent: **24.12.2008**
(21) Application number: 04798729.2
(22) Date of filing: 19.11.2004
(51) Int. Cl.: C07D 498/04, A61K 31/424, A61P 25/00

(54) **PREPARATION OF 1-AZA-2-OXA-DIBENZO [e,h] AZULENES AND THEIR USE FOR THE MANUFACTURE OF PHARMACEUTICAL FORMULATIONS FOR THE TREATMENT AND PREVENTION OF CENTRAL NERVOUS SYSTEM DISEASES AND DISORDERS**
DARSTELLUNG VON 1-AZA-2-OXADIBENZO[e,h]AZULENEN UND DEREN VERWENDUNG BEI DER HERSTELLUNG PHARMAZEUTISCHER FORMULIERUNGEN ZUR BEHANDLUNG UND PRÄVENTION VON KRANKHEITEN UND ERKRANKUNGEN DES ZENTRALEN NERVENSYSTEMS
PREPARATION DE 1-AZA-2-OXA-DIBENZO [e, h]AZULENES ET LEUR UTILISATION DANS LA FABRICATION DE FORMULATIONS PHARMACEUTIQUES UTILISEES DANS LE TRAITEMENT ET LA PREVENTION DES TROUBLES ET MALADIES DU SYSTEME NERVEUX CENTRAL

(30) Priority: 21.11.2003 HR 20030953
(43) Date of publication of application: 09.08.2006
(73) Proprietor: GlaxoSmithKline istrazivacki centar Zagreb d.o.o., 10000 Zagreb (HR)
(72) Inventor: MERCEP, Mladen, 10000 Zagreb (HR); MESIC, Milan, 100000 Zagreb (HR); PESIC, Dijana, 22000 Sibenik (HR); DZAPO, Iva, 10410 Velika Gorica (HR)
(74) Representative: Lawrence, Geoffrey Mark Prouse
(86) International application number: PCT/HR2004/000050
(87) International publication number: WO 2005/049623

(56) References cited:
- EP-A- 0 063 525
- WO-A-96/14320
- US-A- 4 145 434
- US-B1- 6 288 058

## Description

The present invention relates to compounds from the group of 1-aza-2-oxa-dibenzo[*e*,*h*]azulenes, their pharmacologically acceptable salts and solvates, processes and intermediates for the preparation thereof and to the use thereof for the manufacture of a pharmaceutical composition for the treatment and prevention of diseases, damages and disorders of the central nervous system (CNS) caused by disorders of the neurochemical equilibrium of biogenic amines or other neurotransmitters.

Irregularities in the steady state of biogenic amines (serotonin, norepinephrine, dopamine) and of other neurotransmitters and their receptors that are part of the central neurotransmitter system in CNS may be the cause of various mental diseases, damages and disorders (e.g. depression, schizophrenia, manic behaviour and similar). Pathological changes in CNS caused by disorders of neurotransmitter concentration may occur due to an unbalanced (too big or too small) synthesis, irregularities in storing, releasing, metabolizing and/or reabsorption of biogenic amines and/or certain neurotransmitters.

The results of investigations directed to the understanding of pathogenesis of mental disorders have shown that a disorder in the serotonin equilibrium plays an important role in various diseases. The monoamine-deficiency hypothesis was one of the first explanations, wherein the symptoms of depression were connected to a reduction in the neurotransmission of monoamines, especially serotonin (5-HT) and noradrenaline, which was also confirmed by neurochemical tests as well as by a successful treatment of the patients with substances increasing monoaminergic neurotransmission (Expert Opin. Investig. Drugs 2003, 12, 531-543). In addition to the serotonergic and noradrenergic systems, a very important role in CNS function disorders is also played by the dopaminergic system. The understanding of the exact role and of the interactions of these neurotransmitter systems is made rather difficult by the great number of receptor subtypes and their pharmacological complexity. Thus, it has been observed that e.g. dopaminergic neurotransmission is regulated by 5-HT_{2A} receptors (L. G. Spampinato, J. Neurochem. 2000, 74, 693-701) and hence 5-HT_{2A} receptors may also be the target receptors in treating diseases and disorders, in whose pathology an important role is played by a disorder of the function of the dopaminergic system (psychoses and various addictions).

Glutamate receptors play a vital role in the mediation of excitatory synaptic transmission as one of the major excitatory neurotransmitters in central nervous system (CNS). It is widely accepted that σ1 receptor ligands can modulate neurotransmission mediated by central neurotransmitter systems, including glutamatergic/NMDA (F.P. Monnet, G. Debonnel, J.-L. Junien, C. de Montigny, Eur. J. Pharmacol., 1990, 179, 441-445). Many pharmacological and physiological actions have been attributed to σ1 receptor. These include the regulation of IP3 receptors and calcium signaling at the endoplasmic reticulum, mobilization of cytoskeletal adaptor proteins, modulation of nerve growth factor-induced neurite sprouting, modulation of neurotransmitter release and neuronal firing, modulation of potassium channels as a regulatory subunit, alteration of psychostimulant-induced gene expression, and blockade of spreading depression. Behaviorally, σ1 receptor is involved in learning and memory, psychostimulant-induced sensitization, cocaine-induced conditioned place preference, schizophrenia and pain perception. Thus, it is hypothesized that σ1 receptor, at least in part, is intracellular amplifier creating a supersensitized state for signal transduction in the biological system.

For treatment of pathological CNS disorders and particularly in the therapy of mental disorders a significant role as the most frequently applied medicines is given to substances that, according to their structure, are polycyclic compounds (benzodiazepines, tricyclic and tetracyclic antidepressants, monoamino oxidase (MAO) inhibitors, selective inhibitors of serotonin reabsorption etc.).

A new area in pharmacotherapy was opened by introducing the novel tetracyclic antidepressant mianserin (Claghom, J.; Lesem, M. D. Prog. Drug Res. 1996, 46, 243-262; Sperling, W.; Demling, J. Drugs Today 1997, 33, 95-102). Numerous tetracyclic derivatives showing pharmacological action in the treatment of the disorders of the neurochemical equilibrium in CNS are disclosed in the literature. WO 99/19317, WO 97/38991 and US 6,511,976 describe the manufacture of tetracyclic derivatives containing tetrahydrofuran ring and the use thereof as substances having antipsychotic, cardiovascular and gastrokinetic actions. US 4,145,434 discloses the manufacture of dibenzo(cyclohepta-, oxepino-, thiepino-)pyrrolidine and dibenzopyrrolidinoazepine derivatives as well as the use thereof as substances having a potential CNS action. The manufacture and an antidepressive action of some 1,2-diazadibenzoazepines are disclosed in EP 0063525. The manufacture and a potential anxiolytic action of some tetracyclic isooxazolidine derivatives are disclosed as well (Drugs Fut. 2002, 27, Suppl. A: C41; Drugs Fut. 2002, 27, Suppl. A: P182, WO 96/14320, WO 96/14321). The introduction of a piperidine ring into a tetracyclic structure containing an oxepine ring resulted in the formation of the molecule Org-4428 showing an antidepressive action (Sperling, W.; Demling, J. Drugs Today 1997, 33, 95-102). The molecule Org-5222 contains a pyrrolidine ring fused to an oxepine nucleus and is described as a potential anxiolytic and antipsychotic (Sperling, W.; Demling, J. Drugs Today 1997, 33, 95-102). Some derivatives of 1,3-diaza-dibenzo[*e*,*h*]azulenes and salts thereof as a novel class of compounds with antiinflammatory action are known as well (US 3,711,489, US 4,198,421 and CA 967,573).

There are also known 2-substituted dibenzoazulenes of tetrahydro pyrazole class with substituents such as acyl alkyloxycarbonyl, phenyl or substituted phenyls (Gansser C. et al., Ann. Pharm. 1984, 41: 465-471; or Olivera R. et al., Tetrahedron Letters, 2000, 41: 4353-4356, 4357-4360). Further, there are known examples of dibenzoazepines of pyrazole and isoxazole class substituted with alkyl (Kawashiha K. Takeda, Kenkyusho Ho, 1978, 37: 6-11, Fishou D. et al., Tetrahedron 1984, 40: 5121-5133), phenyl or substituted phenyl (FR 2,504,140, EP 0063525).

However, art known medicines used in therapy of pathological CNS disorders and particularly in the therapy of mental disorders are associated with a wide range of adverse effects. There is thus a need for a safe and effective treatment of diseases and disorders of CNS.

New compounds from the class of 1-aza-2-oxa-dibenzo[*e,h*]azulenes represented by the formula **I,** representing the subject of the present invention, their pharmacologically acceptable salts and solvates and pharmaceutical compositions comprising them have hithero not been described.

Moreover, no compound representing the subject matter of the present invention has been described as effective in the treatment of diseases and disorders of CNS. Consequently, the use of 1-aza-2-oxa-dibenzo[*e,h*]azulenes and of their pharmaceutically acceptable salts and solvates for the manufacture of a pharmaceutical composition for the treatment and prevention of diseases, damages and disorders of the central nervous system caused by disorders of neurochemical equilibrium has hitherto been neither disclosed nor suggested.

The compounds from the class of 1-aza-2-oxa-dibenzo[*e,h*]azulenes represented by the formula **I,** differ structurally from the art-known tetracyclic compounds acting upon CNS by an unsaturated tetracyclic structure since they contain an isoxazole ring as the fourth ring, whereas the art-known tetracyclic compounds acting upon CNS (WO 99/19317, WO 97/38991; Sperling, W.; Demling, J. Drugs Today 1997, 33, 95-102) contain at least one saturated ring in their structure, and are further distinguished by valuable pharmacological and physicochemical properties.
The compounds represented by the formula **I,** which are the subject matter of the present invention, isomeric forms of such compounds, their pharmaceutically acceptable salts and solvates and pharmaceutical composition comprising them are not believed to have been previously described. Moreover, no compound representing the subject matter of the present invention has been described as effective in the treatment of diseases and disorders of CNS.
The present invention relates to the compounds from the class of 1-aza-2-oxa-dibenzo[*e,h*]azulenes of the general formula I: 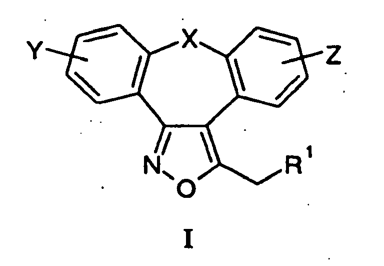 wherein
- X: means CH₂ or a heteroatom selected from the group consisting of O, S, S(=O), S(=O)₂ and NR^{a}, wherein R^{a} is hydrogen or a substituent selected from the group consisting of C₁-C₃-alkyl, C₁-C₃-alkanoyl, C₁-C₇-alkoxycarbonyl, C₇-C₁₀-arylalkyloxycarbonyl, C₇-C₁₀-aroyl, C₇-C₁₀-arylalkyl, C₃-C₇-alkylsilyl and C₅-C₁₀-alkylsilylalkyloxyalkyl;
- Y and Z: independently from each other mean one or more identical or different substituents linked to any available carbon atom selected from the group consisting of hydrogen, halogen , C₁-C₄-alkyl, C₂-C₄-alkenyl, C₂-C₄-alkinyl, halo-C₁-C₄-alkyl, hydroxy, C₁-C₄-alkoxy, trifluoromethoxy, C₁-C₄-alkanoyl, amino, amino-C₁-C₄-alkyl, C₁-C₄-alkylamino, N-(C₁-C₄-alkyl)amino, *N,N-*di(C₁-C₄-alkyl)amino, thiol, C₁-C₄-alkylthio, sulfonyl, C₁-C₄-alkylsulfonyl, sulfinyl, C₁-C₄-alkylsulfinyl, carboxy, C₁-C₄-alkoxycarbonyl, cyano and nitro;
- R¹: means hydrogen, halogen, C₁-C₇-alkyl optionally substituted with one, two, three or more substituents selected from the group consisting of halogen atom, hydroxy, C₁-C₄ alkoxy, thiol, C₁-C₄ alkylthio, amino, N-(C₁-C₄) alkylamino, N,N-di(C₁-C₄-alkyl)-amino, sulfonyl, C₁-C₄ alkylsulfonyl, sulfinyl and C₁-C₄ alkylsulfinyl; C₂-C₇-alkenyl optionally substituted with one, two, three or more halogen atoms; C₂-C₇-alkinyl; monocyclic or bicyclic aryl group having from 6 to 10 carbon atoms and alternating double bonds and said group can be optionally substituted with one or two substituents selected from the group consisting of fluoro, chloro, C₁-C₄ alkyl, cyano, nitro, hydroxy, C₁-C₄ alkoxy, thiol, C₁-C₄ alkylthio, amino, *N*-(C₁-C₄) alkylamino, *N,N*-di(C₁-C₄-alkyl)amino, sulfonyl, C₁-C₄ alkylsulfonyl, sulfinyl, C₁-C₄ alkylsulfinyl and can be linked to the rest of the molecule by any available carbon atom via direct bond or via C₁-C₄ alkylene group; monocyclic or bicyclic heteroaryl having the meaning of aromatic and partially aromatic groups of a monocyclic or bicyclic ring with 4 to 12 carbon atoms and at least one of them being heteroatom selected from the group consisting of O, S and N wherein available carbon or nitrogen represent the binding site of the group to the rest of the molecule either via direct bond or via C₁-C₄ alkylene group and where said heteroaryl can be optionally substituted with fluoro, chloro, C₁-C₄ alkyl, cyano, nitro, hydroxy, C₁-C₄ alkoxy, thiol, C₁-C₄ alkylthio, amino, *N*-(C₁-C₄) alkylamino, *N,N*-di(C₁-C₄-alkyl)-amino, sulfonyl, C₁-C₄ alkylsulfonyl, sulfinyl, C₁-C₄
alkylsulfinyl; five-membered or six-membered fully saturated or partly unsaturated heterocycle group containing at least one hetero atom selected from the group consisting of O, S and N wherein available carbon or nitrogen represent the binding site of the group to the rest of the molecule either via direct bond or via C₁-C₄ alkylene group and where said heterocycle can be optionally substituted with fluoro, chloro, C₁-C₄ alkyl, cyano, nitro, hydroxy, C₁-C₄ alkoxy, thiol, C₁-C₄ alkylthio, amino, *N*-(C₁-C₄) alkylamino, *N,N*-di(C₁-C₄-alkyl)-amino, sulfonyl, C₁-C₄ alkylsulfonyl, sulfinyl, C₁-C₄ alkylsulfinyl; hydroxy; hydroxy-C₂-C₇-alkenyl; hydroxy-C₂-C₇-alkinyl; C₁-C₇-alkoxy; thiol; thio-C₂-C₇-alkenyl; thio-C₂-C₇-alkinyl; C₁-C₇-alkylthio; amino; *N*-(C₁-C₇-alkyl)amino; *N,N*-di(C₁-C₇-alkyl)amino; C₁-C₇-alkylamino; amino-C₂-C₇-alkenyl; amino-C₂-C₇-alkinyl; amino-C₁-C₇-alkoxy; C₁-C₇-alkanoyl; C₇-C₁₀-aroyl; oxo-C₁-C₇-alkyl; C₁-C₇-alkanoyloxy; carboxy; an optionally substituted C₁-C₇-alkyloxycarbonyl; an optionally substituted C₇-C₁₀-aryloxycarbonyl; carbamoyl; *N*-(C₁-C₇-alkyl)carbamoyl; *N,N*-di(C₁-C₇-alkyl)carbamoyl; cyano; cyano-C₁-C₇-alkyl; sulfonyl; C₁-C₇-alkylsulfonyl; sulfinyl; C₁-C₇-alkylsulfinyl; nitro;
or a substituent represented with the formula **II:** 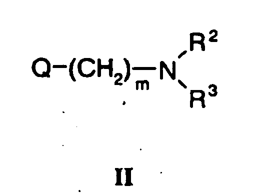 wherein
- R² and R³: simultaneously or independently from each other have the meaning of hydrogen, C₁-C₄-alkyl, aryl having the meaning as defined above; or together with N have the meaning of optionally substituted heterocycle or heteroaryl wherein heterocycle relates to five-membered or six-membered fully saturated or partly unsaturated heterocycle group containing at least one hetero atom selected from the group consisting of O, S and N and where said heterocycle can be optionally substituted with one or two substituents which are selected from halogen, C₁-C₄ alkyl, cyano, nitro, hydroxy, C₁-C₄ alkoxy, thiol, C₁-C₄ alkylthio, amino, *N*-(C₁-C₄) alkylamino, *N,N*-di(C₁-C₄-alkyl)-amino, sulfonyl, C₁-C₄ alkylsulfonyl, sulfinyl, C₁-C₄ alkylsulfinyl and heteroaryl relates to aromatic and partially aromatic groups of a monocyclic or bicyclic ring with 4 to 12 carbon atoms and at least one of them being heteroatom selected from the group consisting of O, S and N and where said heteroaryl can be optionally substituted with one or two substituents which are selected from halogen, C₁-C₄ alkyl, cyano, nitro, hydroxy, C₁-C₄ alkoxy, thiol, C₁-C₄ alkylthio, amino, *N*-(C₁-C₄) alkylamino , *N,N-*di(C₁-C₄-alkyl)-amino, sulfonyl, C₁-C₄ alkylsulfonyl, sulfinyl, C₁-C₄ alkylsulfinyl;
- m: has the meaning of an integer from 1 to 3;
- Q: has the meaning of oxygen, sulfur or NH;
and to their pharmaceutically acceptable salts and solvates, as well as to pharmaceutical compositions containing one or more of the foregoing compounds in an amount effective to treat and prevent diseases, damages and disorders of the central nervous system caused by disorders of neurochemical equilibrium of biogenic amines or other neurotransmitters.

When X has the meaning of NR^{a}, R^{a} relates to hydrogen or a group selected from C₁-C₃-alkyl (preferably methyl or ethyl), C₁-C₃-alkanoyl (preferably formyl or acetyl), C₁-C₇-alkoxycarbonyl (preferably methoxycarbonyl or *tert*-butoxycarbonyl), C₇-C₁₀-arylalkyloxycarbonyl (preferably benzyloxycarbonyl), C₇-C₁₀-aroyl (preferably benzoyl), C₇-C₁₀-arylalkyl (preferably benzyl), C₃-C₇-alkylsilyl (preferably trimethylsilyl) or C₅-C₁₀-alkylsilylalkoxyalkyl (preferably trimethylsilylethoxymethyl).

When R² and R³ together with N have the meaning of heteroaryl or heterocycle, this means that such heteroaryls or heterocycles have at least one carbon atom replaced by a nitrogen atom through which the groups are linked to the rest of the molecule. Examples of such groups are morpholin-4-yl, piperidin-1-yl, pyrrolidin-1-yl, imidazol-1-yl or piperazin-1-yl.

In one embodiment of the present invention preferred compounds of formula I are those wherein X represents O or S.

In another embodiment of the present invention preferred compounds of formula I are those wherein Y and Z independently from each other mean one or more identical or different substituents linked to any available carbon atom selected from the group consisting of hydrogen, fluorine, chlorine, bromine, C₁-C₄-alkyl (preferably methyl, ethyl, propyl or isopropyl), halo-C₁-C₄-alkyl (preferably trifluoromethyl), hydroxy, C₁-C₄-alkoxy (preferably methoxy), trifluoromethoxy, C₁-C₄-alkanoyl (preferably formyl or acetyl), amino, amino-C₁-C₄-alkyl (preferably aminomethyl), *N*-(C₁-C₄-alkyl)amino (preferably *N*-methyl or *N*-ethyl), *N,N*-di(C₁-C₄-alkyl)amino(preferably dimethylamino or diethylamino), thiol, C₁-C₄-alkylthio (preferably methylthio), cyano and nitro.

In yet another embodiment of the present invention preferred compounds of formula I are those wherein R¹ has the meaning of hydrogen, halogen, C₁-C₇-alkyl optionally substituted with one, two, three or more substituents selected from the group consisting of halogen atom, hydroxy, C₁-C₄ alkoxy, thiol, C₁-C₄ alkylthio, amino, N-(C₁-C₄) alkylamino and *N,N*-di(C₁-C₄-alkyl)-amino; monocyclic or bicyclic aryl group having from 6 to 10 carbon atoms and alternating double bonds and said group can be optionally substituted with one or two substituents selected from the group consisting of fluoro, chloro, C₁-C₄ alkyl, cyano, nitro, hydroxy, C₁-C₄ alkoxy, thiol, C₁-C₄ alkylthio, amino, *N*-(C₁-C₄) alkylamino and *N,N*-di(C₁-C₄-alkyl)-amino and can be linked to the rest of the molecule by any available carbon atom via direct bond or via C₁-C₄ alkylene group; monocyclic or bicyclic heteroaryl having the meaning of aromatic and partially aromatic groups of a monocyclic or bicyclic ring with 4 to 12 carbon atoms and at least one of them being heteroatom selected from the group consisting of O, S and N wherein available carbon or nitrogen represent the binding site of the group to the rest of the molecule either via direct bond or via C₁-C₄ alkylene group and where said heteroaryl can be optionally substituted with fluoro, chloro, C₁-C₄ alkyl, cyano, nitro, hydroxy, C₁-C₄ alkoxy, thiol, C₁-C₄ alkylthio, amino, *N*-(C₁-C₄) alkylamino and *N,N*-di(C₁-C₄-alkyl)-amino; five-membered or six-membered fully saturated or partly unsaturated heterocycle group containing at least one hetero atom selected from the group consisting of O, S and N wherein available carbon or nitrogen represent the binding site of the group to the rest of the molecule either via direct bond or via C₁-C₄ alkylene group and where said heterocycle can be optionally substituted with fluoro, chloro, C₁-C₄ alkyl, cyano, nitro, hydroxy, C₁-C₄ alkoxy, thiol, C₁-C₄ alkylthio, amino, *N*-(C₁-C₄) alkylamino and *N,N*-di(C₁-C₄-alkyl)-amino; hydroxy; C₁-C₇-alkoxy; thiol; C₁-C₇-alkylthio; amino; *N*-(C₁-C₇-alkyl)amino; *N,N*-di(C₁-C₇-alkyl)amino; amino-C₁-C₇-alkoxy; C₁-C₇-alkanoyl; C₇-C₁₀-aroyl; C₁-C₇-alkanoyloxy; an optionally substituted C₁-C₇-alkyloxycarbonyl; an optionally substituted C₇-C₁₀-aryloxycarbonyl; carbamoyl; *N*-(C₁-C₇-alkyl)carbamoyl; *N,N-*di(C₁-C₇-alkyl)carbamoyl; cyano; cyano-C₁-C₇-alkyl; nitro;
or a substituent represented with the formula **II:** 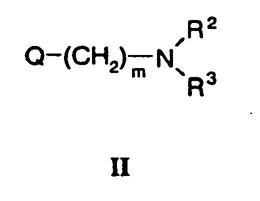 wherein
- R² and R³: simultaneously or independently from each other have the meaning of hydrogen, C₁-C₄-alkyl, aryl having the meaning as described above; or together with N have the meaning of heterocycle or heteroaryl selected from the group consisting of morpholine-4-yl, piperidine-1-yl, pyrrolidine-1-yl, imidazole-1-yl and piperazine-1-yl;
- m: has the meaning of an integer from 1 to 3;
- Q: has the meaning of oxygen.

In another embodiment of the present invention, preferred compounds of formula I are those wherein Y represents hydrogen or chlorine and Z represents hydrogen.

In yet another embodiment of the present invention, preferred compounds of formula I are those wherein R¹ represents CH₃, CH₂Br, CH₂OH or a substituent of formula II: 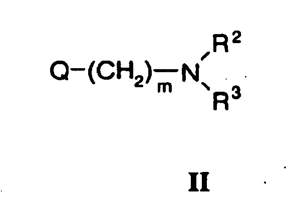 wherein R², R³, Q and m have the above defined meanings.

More specifically preferred are compounds wherein the symbol m has the meaning of 2 or 3.

In yet another embodiment of the present invention the specifically preferred compounds of formula **I** are:
3-methyl-2-oxa-8-thia-1-aza-dibenzo[*e,h*]azulene;
11-chloro-3-methyl-2-oxa-8-thia-1-aza-dibenzo[*e,h*]azulene;
3-methyl-2,8-dioxa-1-aza-dibenzo[*e,h*]azulene;
3-bromomethyl-2-oxa-8-thia-1-aza-dibenzo[*e,h*]azulene;
3-bromomethyl-11-chloro-2-oxa-8-thia-1-aza-dibenzo[*e,h*]azulene;
3-bromomethyl-2,8-dioxa-1-aza-dibenzo[*e,h*]azulene;
dimethyl-[2-(2-oxa-8-thia-1-aza-dibenzo[*e,h*]azulen-3-ylmethoxy)-ethyl]-amine;
dimethyl-[3-(2-oxa-8-thia-1-aza-dibenzo[*e,h*]azulen-3-ylmethoxy)-propyl]-amine;
dimethyl-[2-(11-chloro-2-oxa-8-thia-1-aza-dibenzo[*e,h*]azulen-3-ylmethoxy)-ethyl]-amine;
dimethyl-[3-(11-chloro-2-oxa-8-thia-1-aza-dibenzo[*e,h*]azulen-3-ylmethoxy)-propyl]-amine;
dimethyl-[2-(2,8-dioxa-1-aza-dibenzo[*e,h*]azulen-3-ylmethoxy)-ethyl]-amine; and
dimethyl-[3-(2,8-dioxa-1-aza-dibenzo[*e,h*]azulen-3-ylmethoxy)-propyl]-amine.

The term "halo", "hal" or "halogen" relates to a halogen atom which may be fluorine, chlorine, bromine or iodine.

The term "alkyl" relates to alkyl groups with the meaning of alkanes wherefrom radicals are derived, which radicals may be straight, branched or cyclic or a combination of straight and cyclic ones and branched and cyclic ones. The preferred straight or branched alkyls are e.g. methyl, ethyl, propyl, *iso*-propyl, butyl, *sec*-butyl and *tert*-butyl. The preferred cyclic alkyls are e.g. cyclopentyl or cyclohexyl.

The term "haloalkyl" relates to alkyl groups which must be substituted with at least one halogen atom. The most frequent haloalkyls are e.g. chloromethyl, dichloromethyl, trifluoromethyl or 1,2-dichloropropyl.

The term "alkenyl" relates to alkenyl groups having the meaning of hydrocarbon radicals, which may be straight, branched or cyclic or are a combination of straight and cyclic ones or branched and cyclic ones, but having at least one carbon-carbon double bond. The most frequent alkenyls are ethenyl, propenyl, butenyl or cyclohexenyl.

The term "alkinyl" relates to alkinyl groups having the meaning of hydrocarbon radicals, which are straight or branched and contain at least one and at most two carbon-carbon triple bonds. The most frequent alkinyls are e.g. ethinyl, propinyl or butinyl.

The term "alkoxy" relates to straight or branched chains of alkoxy group. Examples of such groups are methoxy, propoxy, prop-2-oxy, butoxy, but-2-oxy or methylprop-2-oxy.

The term "aryl" relates to groups having the meaning of an aromatic ring, e.g. phenyl, as well as to fused aromatic rings. Aryl contains one ring with at least 6 carbon atoms or two rings with totally 10 carbon atoms and with alternating double (resonant) bonds between carbon atoms. The most freqently used aryls are e.g. phenyl or naphthyl. In general, aryl groups may be linked to the rest of the molecule by any available carbon atom via a direct bond or via a C₁-C₄-alkylene group such as methylene or ethylene.

The term "heteroaryl" relates to groups having the meaning of aromatic and partially aromatic groups of a monocyclic or bicyclic ring with 4 to 12 atoms, at least one of them being a hetero atom such as O, S or N, and the available nitrogen atom or carbon atom is the binding site of the group to the rest of the molecule either via a direct bond or via a C₁-C₄-alkylene group defined earlier. Examples of this type are thiophenyl, pyrrolyl, imidazolyl, pyridinyl, oxazolyl, thiazolyl, pyrazolyl, tetrazolyl, pyrimidinyl, pyrazinyl, quinolinyl or triazinyl.

The term "heterocycle" relates to five-membered or six-membered, completely saturated or partly unsaturated heterocyclic groups containing at least one hetero atom such as O, S or N, and the available nitrogen atom or carbon atom is the binding site of the group to the rest of the molecule either via a direct bond or via a C₁-C₄-alkylene group defined earlier. The most frequent examples are morpholinyl, piperidyl, piperazinyl, pyrrolidinyl, pyrazinyl or imidazolyl.

The term "alkanoyl" group relates to straight chains of acyl group such as formyl, acetyl or propanoyl.

The term "aroyl" group relates to aromatic acyl groups such as benzoyl.

The term "optionally substituted alkyl" relates to alkyl groups which may be optionally additionally substituted with one, two, three or more substituents. Such substituents may be halogen atom (preferably chlorine or fluorine), hydroxy, C₁-C₄-alkoxy (preferably methoxy or ethoxy), thiol, C₁-C₄-alkylthio (preferably methylthio or ethylthio), amino, *N*-(C₁-C₄-alkyl)amino (preferably *N*-methylamino or N-ethylamino), *N,N*-di(C₁-C₄-alkyl)amino (preferably dimethylamino or diethylamino), sulfonyl, C₁-C₄-alkylsulfonyl (preferably methylsulfonyl or ethylsulfonyl), sulfinyl, C₁-C₄-alkylsulfinyl (preferably methylsulfinyl).

The term "optionally substituted alkenyl" relates to alkenyl groups optionally additionally substituted with one, two or three halogen atoms. Such substituents may be e.g. 2-chloroethenyl, 1,2-dichloroethenyl or 2-bromo-propen-1-yl.

The term "optionally substituted aryl, heteroaryl or heterocycle" relates to aryl, heteroaryl or heterocyclic groups which may be optionally additionally substituted with one or two substituents. The substituents may be halogen (preferably chlorine or fluorine), C₁-C₄-alkyl (preferably methyl, ethyl or isopropyl), cyano, nitro, hydroxy, C₁-C₄-alkoxy (preferably methoxy or methoxy), thiol, C₁-C₄-alkylthio (preferably methylthio or ethylthio), amino, *N*-(C₁-C₄)alkylamino (preferably N-methylamino or N-ethylamino), *N,N*-di(C₁-C₄-alkyl)amino (preferably *N,N*-dimethylamino or *N,N-*diethylamino), sulfonyl, C₁-C₄-alkylsulfonyl (preferably methylsulfonyl or ethylsulfonyl), sulfinyl, C₁-C₄-alkylsulfinyl (preferably methylsulfinyl).

Depending upon the nature of particular substituents, the compounds of the formula I may have geometric isomers and one or more chiral centres so that there can exist enantiomers or diastereoisomers. The present invention also relates to the use of such isomers and mixtures thereof, including racemates.

The present invention also relates to all possible tautomeric forms of particular compounds of the formula **I.**

Whenever used hereinafter, the term "compounds of formula **I"** or "compounds of the present invention" is meant to also include the pharmaceutically acceptable addition salts and solvates.

The term "salts" can include acid addition salts or addition salts of free bases. Examples of acids which may be employed to form pharmaceutically acceptable acid addition salts include but are not limited to salts derived from nontoxic inorganic acids such as nitric, phosphoric, sulfuric, or hydrobromic, hydroiodic, hydrofluoric, phosphorous, as well as salts derived from nontoxic organic acids such as aliphatic mono- and dicarboxylic acids, phenyl-substituted alkanoic acids, hydroxyl alkanoic acids, alkanedioic acids, aromatic acids, aliphatic and aromatic sulfonic acids, and acetic, maleic, succinic, or citric acids. Examples of such salts include napadisylate, besylate, sulfate, pyrosulfate, bisulfate, sulfite, bisulfite, nitrate, phosphate, monohydrogenphosphate, dihydrogenphosphate, metaphosphate, pyrophosphate, chloride, bromide, iodide, acetate, trifluoroacetate, propionate, caprylate, isobutyrate, oxalate, malonate, succinate, suberate, sebacate, fumarate, maleate, mandelate, benzoate, chlorobenzoate, methylbenzoate, dinitrobenzoate, phthalate, benzenesulfonate, toluenesulfonate, phenylacetate, citrate, lactate, maleate, tartrate, methanesulfonate. Also contemplated are salts of amino acids such as arginate and gluconate, galacturonate (see, for example, Berge S. M. et al. "Pharmaceutical Salts," J. of Pharma. Sci., 1977; 66:1).

The acid addition salts of said basic compounds are prepared by contacting the free base form with a sufficient amount of the desired acid to produce the salt in the conventional manner. The free base form may be regenerated by contacting the salt form with a base and isolating the free base in the conventional manner. The free base forms differ from their respective salt forms somewhat in certain physical properties such as solubility in polar solvents, but otherwise the salts are equivalent to their respective free base for purposes of the present invention.

Pharmaceutically acceptable base addition salts are formed with metals or amines, such as alkali and alkaline earth metals or organic amines. Examples of metals used as cations are sodium, potassium, magnesium, calcium. Examples of suitable amines are N,N'-dibenzylethylenediamine, chloroprocaine, choline, diethanolamine, dicyclohexylamine, ethylenediamine, N-methylglucamine, and procaine.

The base addition salts of said acidic compounds are prepared by contacting the free acid form with a sufficient amount of the desired base to produce the salt in the conventional manner. The free acid form may be regenerated by contacting the salt form with an acid and isolating the free acid.

Preferred pharmaceutically acceptable salts according to the invention relate to salts of the formula **I** and include e.g. salts with C₁-C₄-alkylhalides (preferably methyl bromide, methyl chloride) (quaternary ammonium salts), with inorganic acids (hydrochloric, hydrobromic, phosphoric, metaphosphoric, nitric or sulfuric acids) or with organic acids (tartaric, acetic, citric, maleic, lactic, fumaric, benzoic, succinic, methane sulfonic or *p*-toluene sulfonic acids).

Pharmaceutically acceptable solvates formed by the compounds represented by formula **I** or their salts relate to hydrates, ethanolates and similar (most frequently hydrates).

The phrase "pharmaceutically acceptable", as used in connection with compositions of the invention, refers to molecular entities and other ingredients of such compositions that are physiologically tolerable and do not typically produce untoward reactions when administered to a mammal (e.g., human). Preferably, as used herein, the term "pharmaceutically acceptable" means approved by a regulatory agency of the Federal or a state government or listed in the U.S. Pharmacopoeia or other generally recognized pharmacopeias for use in mammals, and more particularly in humans.

A further object of the present invention relates to the preparation of the compounds of the formula **I** according to the following processes:
a) condensation of compound **Ia:** 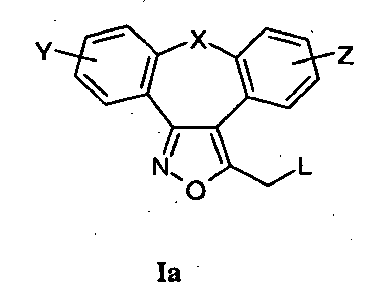 wherein X, Y and Z have the earlier stated meanings, L has the meaning of a leaving group,
   with an optionally selected alcohol, thioalcohol or amine or with a compound of the formula **IIa:** 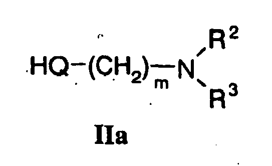 wherein all radicals and symbols have the earlier stated meanings; or
b) condensation of compound of the formula **Ib:** 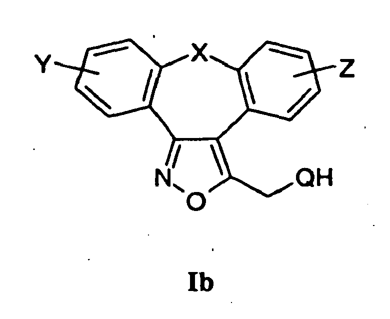
wherein all symbols have the earlier stated meanings, with a compound of the formula **IIb:** 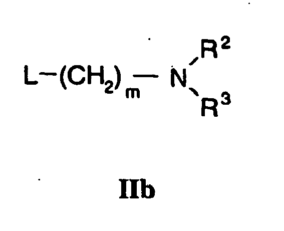 wherein radicals R² and R³ and symbol m have the earlier stated meanings and symbol L has the meaning of a good leaving group. Suitable leaving groups for these reactions include halide (e.g. chloride, bromide or iodide).

### Preparation methods

a) Compounds of the formula **I** according to the present process are prepared by reaction of compounds of the formula **Ia,** wherein L has the meaning of a leaving group, with optionally selected alcohols, thioalcohols or amines, or with compounds of the formula **IIa,** wherein Q has the meaning of oxygen, nitrogen or sulfur. The condensation reactions may be carried out most conveniently according to methods disclosed for the preparation of analogous compounds (Menozzi G et al., J. Heterocyclic Chem., 1997, 34:963-968 or WO 01/87890). The reactions are carried out at a temperature from 20°C to 100°C during 1 to 24 hours in a two-phase system (preferably with 50% NaOH/toluene), sometimes in the presence of a phase transfer catalyst (preferably benzyl triethyl ammonium chloride, benzyl triethyl ammonium bromide, cetyl trimethyl bromide). After the treatment of the reaction mixture, the products formed are isolated by recrystallization or chromatography on a silica gel column.
   The starting compounds of the formula **Ia** (most frequently halides) may be obtained by the reaction sequence represented in Scheme I according to the processes described for analogous compounds (Talley J. J. et al., J. Med. Chem., 2000, 43: 775-777). 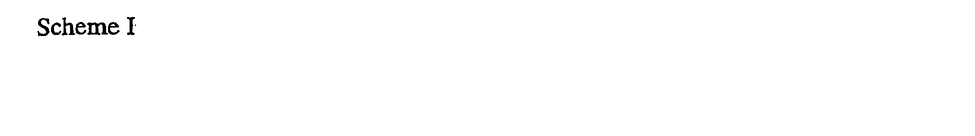 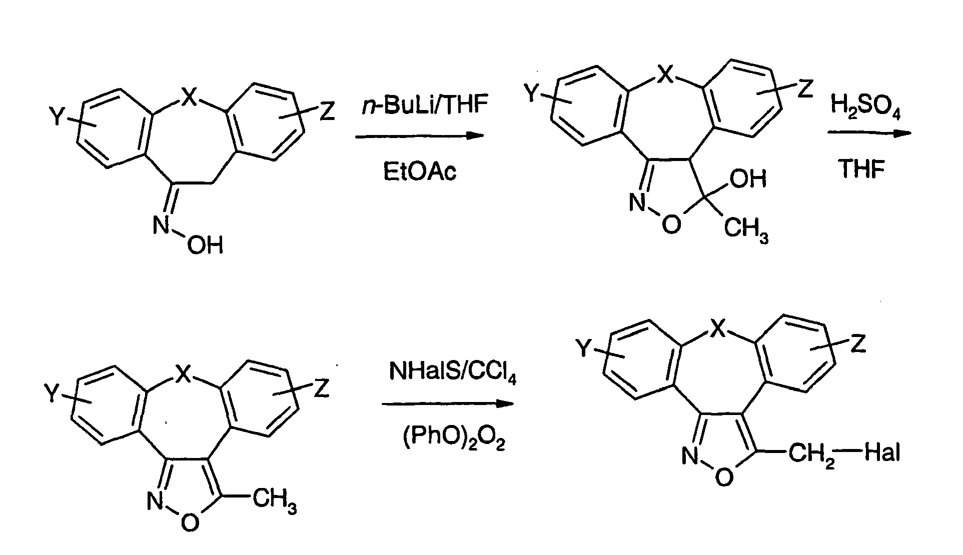
   Hydroxylamines required for the above reaction sequence are compounds known from the literature or are prepared by the action of NH₂OH · HCl upon ketones 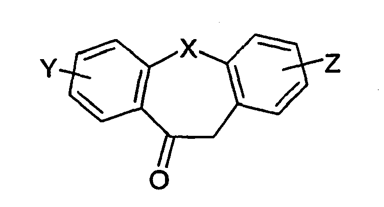 in the presence of NaOAc in an alcohol-aqueous medium.
   The starting alcohols, thioalcohols or the compounds of the formula **IIa** are commercially available substances or are prepared according to methods disclosed for the preparation of analogous compounds.
b) The compounds of the formula **I** may be prepared according to the present process by condensation of compounds of formula **Ib** with optionally selected halides or with compounds of formula **IIb,** wherein L has the meaning of a leaving group. The condensation reactions are reactions of nucleophilic substitution on saturated carbon atom, which are described in the literature and are carried out in an analogous manner as described in method a).

The starting compounds, alcohols of the formula **Ib,** may be obtained by the action of water, ammonia or hydrogen sulfide upon halides of formula **Ia** in a manner disclosed in the literature. The starting optionally selected halides or compounds of the formula **IIb** are already known or are prepared according to methods disclosed for the preparation of analogous compounds.

Besides the above-mentioned reactions, the compounds of the formula **I** may be prepared by the transformation of other earlier prepared compounds of the formula **I** and it is to be understood that the present invention also comprises such compounds and processes. An example of such transformation is a reaction of the aldehyde group with chosen phosphorous ylides resulting in a prolongation of the chain and the formation of an alkenyl substituent with carbonyl or ester groups as disclosed in WO 01/87890. These reactions are carried out in solvents such as benzene, toluene or hexane at elevated temperature (most frequently at boiling temperature of the solvent).

A further general example of transformation is formylation of the compounds of the formula **I** by processes such as e.g. Vilsmeier acylation or reaction of *n*-BuLi and dimethylformamide. The reaction conditions of these processes are known in the literature.

By hydrolysis of the compounds of the formula **I** having nitrile, amide or ester groups, there may be prepared compounds with a carboxyl group, which are suitable intermediates for the preparation of other compounds with novel functional groups such as e.g. esters, amides, halides, anhydrides, alcohols or amines.

Oxidation or reduction reactions are a further possibility of the change of substituents in the compounds of the formula **I.** Most frequently used oxidation agents are peroxides (hydrogen peroxide, *m*-chloroperbenzoic acid or benzoyl peroxide) or permanganate, chromate or perchlorate ions. Thus e.g. by the oxidation of an alcohol group by pyridinyl dichromate or pyridinyl chlorochromate, an aldehyde group is formed, which group may be converted to a carboxyl group by further oxidation.

By a selective oxidation of alkylthio group, alkylsulfinyl or alkylsulfonyl groups may be prepared.

By the reduction of the compounds with a nitro group, the preparation of amino compounds is made possible. The reaction is carried out under usual conditions of catalytic hydrogenation or electrochemically. By catalytic hydrogenation using palladium on carbon, alkenyl substituents may be converted to alkyl ones or nitrile group can be converted to aminoalkyl.

Various substituents of the aromatic structure in the compounds of the formula I may be introduced by standard substitution reactions or by usual changes of individual functional groups. Examples of such reactions are aromatic substitutions, alkylations, halogenation, hydroxylation as well as oxidation or reduction of substituents. Reagents and reaction conditions are known from the literature. Thus e.g. by aromatic substitution a nitro group is introduced in the presence of concentrated nitric acid and sulfuric acid. By using acyl halides or alkyl halides, the introduction of an acyl group or an alkyl group is made possible. The reaction is carried out in the presence of Lewis acids such as aluminum- or iron-trichloride in conditions of Friedel-Craft reaction. By the reduction of the nitro group, an amino group is obtained, which is by a diazotizing reaction converted to a suitable starting group, which may be replaced with one of the following groups: H, CN, OH, Hal.

In order to prevent undesired interaction in chemical reactions, it is often necessary to protect certain groups such as e.g. hydroxy, amino, thio or carboxy. For this purpose a great number of protecting groups may be used [Green TW, Wuts PGH, Protective Groups in Organic Synthesis, John Wiley and Sons, 1999)] and the choice, use and elimination thereof are conventional methods in chemical synthesis.

A convenient protection for amino or alkylamino groups are groups such as e.g. alkanoyl (acetyl), alkoxycarbonyl (methoxycarbonyl, ethoxycarbonyl or *tert-*butoxycarbonyl); arylmethoxycarbonyl (benzyloxycarbonyl), aroyl (benzoyl) or alkylsilyl (trimethylsilyl or trimethylsilylethoxymethyl) groups. The conditions of removing a protecting group depend upon the choice and the characteristics of this group. Thus e.g. acyl groups such as alkanoyl, alkoxycarbonyl or aroyl may be eliminated by hydrolysis in the presence of a base (sodium hydroxide or potassium hydroxide), *tert*-butoxycarbonyl or alkylsilyl (trimethylsilyl) may be eliminated by treatment with a suitable acid (hydrochloric, sulfuric, phosphoric or trifluoroacetic acid), whereas arylmethoxycarbonyl group (benzyloxycarbonyl) may be eliminated by hydrogenation using a catalyst such as palladium on carbon.

The compounds of the present invention are especially effective in treating those diseases and disorders where the neurochemical equilibrium of biogenic amines such as serotonin, norepinephrine and dopamine or other neurotransmitters, preferably glutamate, was disturbed and which may be caused by unbalanced (too big or too small) synthesis, irregularities in storing, releasing, metabolizing and/or reabsorption of a certain neurotransmitter.

The present compounds of the general formula **I** act upon the neurochemical equilibrium by regulating the synthesis, storing, releasing, metabolizing and/or reabsorption of biogenic amines or neurotransmitters and binding to their receptors. It has been found that the compounds of the present invention exhibit a significant binding affinity and have a high degree of selectivity to serotonin receptors, especially to 5-HT_{2A} and 5-HT_{2C}, as well as for σ1 receptor.

In one embodiment of the present invention the compound of formula **I,** or salt, or solvate thereof show binding affinity to 5-HT_{2A} and 5-HT_{2C} serotonin receptors in the concentration expressed as an IC₅₀ value less than 1 µM and having Kᵢ value less than 1 µM.

In another embodiment of the present invention the compound of formula **I,** or salt, or solvate thereof show binding affinity to 5-HT_{2A} serotonin receptor in the concentration expressed as an IC₅₀ value less than about 200 nM and having Kᵢ value less than about 100 nM.

In yet another embodiment of the present invention the compound of formula **I,** or salt, or solvate thereof show binding affinity to 5-HT_{2C} serotonin receptor in the concentration expressed as an IC₅₀ value less than about 200 nM and having Kᵢ value less than about 100 nM.

It has been found that the compounds of the present invention exhibit a significant binding affinity to σ1 receptor.

In one embodiment of the present invention the compound of formula **I,** or salt, or solvate thereof show binding affinity to σ1 receptor in the concentration expressed as an IC₅₀ value less than 1 µM and having Kᵢ value less than 1 µM.

In another embodiment of the present invention the compound of formula **I,** or salt, or solvate thereof show binding affinity to σ1 receptor in the concentration expressed as an IC₅₀ value less than about 200 nM and having Kᵢ value less than about 100 nM.

Since serotonin receptors are crucial in pathophysiology of a series of CNS disorders (directly or indirectly by participating in the activation of some other neurotransmitter e.g. dopamine and/or receptor), the compounds of the present invention may be used for the manufacture of pharmaceutical formulations for the treatment and prevention of diseases, damages and disorders, wherein biogenic amines and their receptors play an important role.

In view of the above explained favourable biological properties of the compounds of the present invention administration of the therapeutically effective amount of a compound of formula **I** provides an effective method of treatment of CNS diseases and disorders associated with fewer side effects due to their improved selectivity towards σ1 receptor and 5-HT_{2A} and 5-HT_{2C} serotonin receptors.

In general, the compounds of the present invention may be used for the manufacture of pharmaceutical formulations that are used as antidepressants, anxiolytics, antipsychotics or as drugs for treating migraine.

Further, the compounds of the present invention may be used for the manufacture of pharmaceutical formulations for the treatment and prevention of diseases and disorders which are the result of disorders of neurochemical equilibrium in the central nervous system such as e.g. depression and modest depression, anxiety, bipolar disorders, sleeping disorders, sexual disorders, psychoses, borderline psychoses, schizophrenia, migraine, personality disorders and obsessive-compulsive disorders, social phobias or panic attacks, organic mental disorders in children, aggression, memory disorders and personality disorders in elderly people, addiction, obesity, bulimia and similar disorders, snoring, premenstrual troubles.

Likewise, these compounds may be used in the treatment and/or prevention of CNS damage caused by trauma, brain stroke, neurodegenerative diseases, cardiovascular disorders such as high blood pressure, thrombosis, infarct and similar diseases as well as in gastrointestinal disorders.

The effective dose of the active substance of the present invention and of a pharmaceutically acceptable salt or solvate thereof depends on the efficacy of the compound of the general formula **I,** on the nature and the severity of the disease and the disorder of CNS as well as on the body weight of the patient treated and may be from 0.001-10 mg/kg body weight. In any case a unit dose for an adult of an average weight of 70 kg is understood to be 0.07-1000 mg of the compound of the general formula **I** or of a pharmaceutically acceptable salt or solvate thereof. A unit dose may be administered once or several times daily, e.g. 2, 3 or 4 times daily, most frequently 1 to 3 times daily.

The present invention more specifically relates to an effective dose of the compounds which bind to serotonin, sigma, adrenergic, dopamine or muscarinic receptors and/or act as inhibitors of reabsorption of one or more biogenic amines (serotonin, dopamine, norepinephrine).

Further, the present invention relates to a pharmaceutical formulation containing an effective non-toxic dose of the compounds of the present invention as well as pharmaceutically acceptable carriers or solvents.

The pharmaceutical formulations are obtained by blending a therapeutically active amount of a certain substance as the active ingredient with a pharmaceutically acceptable carrier, which may have different forms depending on the desired administration route. These pharmaceutical formulations especially relate to oral, sublingual, rectal, percutaneous or parenteral administration route.

Pharmaceutical formulations may be manufactured using conventional pharmaceutical auxiliaries and manufacture routes. Forms for oral administration may be syrups, capsules, tablets where usual solid carriers are inert substances such as lactose, starch, glucose, methylcellulose, magnesium stearate, dicalcium phosphate, mannitol, and usual liquid oral auxiliaries include ethanol, glycerol, water. All auxiliaries may be optionally blended with disintegrants, diluents, granulating agents, wetting agents, binders by using conventional methods. Parenteral forms may be manufactured by using water or some other sterile carrier. When for the manufacture of oral formulations some of the common liquid carriers e.g. water, glycol, oils, alcohols are used, the formulation may be in the form of syrup, emulsion, soft gelatine capsules or sterile injectable liquids e.g. ampoules, or of non-aqueous liquid suspensions. When for the manufacture of oral formulations a solid carrier such as starch, sugar, kaolin, wetting agents, binders, disintegrants is used, the formulation may be in the form of a powder, capsule, tablet, hard gelatine capsules or granules that may be administered in capsules, and the amount of the solid carrier may vary (most frequently from 1 mg to 1 g). Due to their easy use, tablets and capsules are the most convenient oral formulations wherein a solid carrier is used. For parenteral formulations the carrier is mostly sterile water, though other ingredients may be contained therein as well in order to improve solubility. For the manufacture of injectable solutions, odium chloride solution, glucose solution or a mixture thereof is used. Injectable solutions may also contain a component for a delayed release of active component. Convenient oils that may be used for this purpose are e.g. arachic oil, sesame oil, cottonseed oil, corn oil, soybean oil, synthetic glycerol esters of long-chain fatty acids or a mixture of some of said oils. Injectable suspensions may be manufactured in such a way that a suitable liquid carrier used is blended with a suspending agent. In formulations convenient for percutaneous administration, as a carrier there is understood a substance improving the penetration of the active substance and/or a suitable wetting agent, which may be combined with a suitable additive of any provenience, which additives do not cause harmful effects on skin. Said additives may facilitate the skin administration and/or may be used in the manufacture of the desired formulations, which may be applied in various ways e.g. transdermally, spot-on, or in the form of an ointment.

To improve the solubility and/or stability of the present compounds, in pharmacological formulations there may be used α-, β- or γ-cyclodextrins or derivatives thereof, especially hydroxyalkyl substituted cyclodextrins i.e. 2-hydroxypropyl-β-cyclodextrin. Cosolvents such as e.g. alcohols may also improve the solubility and/or stability of the present compounds in various pharmaceutical formulations.

The term "carrier" applied to pharmaceutical compositions of the invention refers to a diluent, excipient, or vehicle with which an active compound is administered..Such pharmaceutical carriers can be sterile liquids, such as water, saline solutions, aqueous dextrose solutions, aqueous glycerol solutions, and oils, including those of petroleum, animal, vegetable or synthetic origin, such as peanut oil, soybean oil, mineral oil, sesame oil. However, since memantine is highly soluble, aqueous solutions are preferred. Suitable pharmaceutical carriers are described in "Remington's Pharmaceutical Sciences" by E.W. Martin, 18th Edition. Particularly preferred for the present invention are carriers suitable for immediate-release, i.e., release of most or all of the active ingredient over a short period of time, such as 60 minutes or less, and make rapid absorption of the drug possible.

A "pharmaceutically acceptable excipient" means an excipient that is useful in preparing a pharmaceutical composition that is generally safe, non-toxic and neither biologically nor otherwise undesirable, and includes an excipient that is acceptable for veterinary use as well as human pharmaceutical use. A "pharmaceutically acceptable excipient" as used in the present application includes both one and more than one such excipient.

"Treating" or "treatment" of a state, disorder or condition includes:
(1) preventing or delaying the appearance of clinical symptoms of the state, disorder or condition developing in a mammal that may be afflicted with or predisposed to the state, disorder or condition but does not yet experience or display clinical or subclinical symptoms of the state, disorder or condition,
(2) inhibiting the state, disorder or condition, i.e., arresting or reducing the development of the disease or at least one clinical or subclinical symptom thereof, or
(3) relieving the disease, i.e., causing regression of the state, disorder or condition or at least one of its clinical or subclinical symptoms.

The benefit to a subject to be treated is either statistically significant or at least perceptible to the patient or to the physician.

A "therapeutically effective amount" means the amount of a compound that, when administered to a mammal for treating a state, disorder or condition, is sufficient to effect such treatment. The "therapeutically effective amount" will vary depending on the compound, the disease and its severity and the age, weight, physical condition and responsiveness of the mammal to be treated.

Dosages and administration regimen can be adjusted depending on the age, sex, physical condition as well as the benefit acchieved by applying the compounds of the present invention and the side effects in the patient or the mammalian subject to be treated and the judgement of the physician, as is appreciated by those skilled in the art.

The term host or subject in need thereof as used herein refers to a mammal preferably a human.

The effect of the compounds of the present invention on the neurochemical steady state was determined by *in vitro* investigations such as a radionuclide-marked radioligand binding assay for 5-HT_{2A} (Bonhaus D. W. Br. J. Pharmacol. 1995, 115:622; Saucier C. J. Neurochem. 1997, 68:1998) and 5-HT_{2C} receptors (Wolf W. A. J. Neurochem. 1997, 69:1449), *in vitro* binding assay for σ1 receptor (Thomson W. and Donn R. Arthritis Res. 2002, 4: 302-306) and by *in vivo* investigations in a tail suspension test (Vogel H. G. and Vogel W. H. Drug Discovery and Evaluation Pharmacological Assays, Springer 1997, 304), in amphethamine-induced hyperlocomotion in mice (Millan M.J. et al, 1998 J Pharmacol. Exp. Ther. 287: 167-186), in a forced swim test in mice (Porsolt R. D. et al. Arch. Int. Pharmacodyn. 1977, 229:327-336), in meta-chlorophenyl piperazine (m-CPP) test on rats (Drug Dev. Res. 1989, 18:119-144), and in apomorphine, tryptamine, norepinephrine (ATN) test in rats (Arch. Int. Pharmacodyn. 1977, 227:238-253).

### In vitro method for determining affinity for binding to 5-HT_{2A} and 5-HT_{2C} receptors

A small concentration of a radioligand having a great affinity for binding to a receptor was incubated with a tissue sample enriched with a certain receptor (1-5 mg of tissue) in a buffered medium (0.2-5 mL). Recombinant human HT_{2A} and HT_{2C} receptors were expressed in CHO-K1 or COS-7 cells and were also used for competitive binding. During incubation the radioligand bound to the receptor. When a binding balance was achieved, the receptors to which the radioligand was bound were separated from those to which said ligand was not bound, and the radioactivity of the receptor/radioligand complex was measured. The interaction of the tested compounds with receptors was tested in competitive binding experiments. Various concentrations of tested compounds were added to the incubation mixture containing a prepared tissue enriched with corresponding receptors and the radioligand. The radioligand binding was inhibited by the test compounds proportionally to the affinity of a certain compound for the receptor and to the concentration of the compound.

The radioligand used for the determination of binding to 5-HT_{2A} receptor was [³H]-ketanserin and the tissue used was human cortex or recombinant 5-HT_{2A} receptor expressed in CHO-K1.

The radioligand used for the determination of binding to 5-HT_{2C} receptor was [³H]-mesulergine and the tissue used was choroid plexus or recombinant 5-HT_{2C} receptor expressed in CHO-K1 cells.

Compounds showing IC₅₀ and Kᵢ in concentrations lower than 1 µM, were considered to be active.
Compounds: 3-methyl-2-oxa-8-thia-1-aza-dibenzo[e,h]azulene and dimethyl-[3-(2-oxa-8-thia-1-aza-dibenzo[e,h]azulen-3-ylmethoxy)-propyl]-amine showed binding affinity to 5-HT_{2A} and 5-HT_{2C} serotonin receptors expressed as IC₅₀ value less than 200 nM and Ki value less than 100 nM.
It is anticipated that similar results will be observed for other compounds of the invention.

### In vitro method for determining binding affinity to σ1 receptor

Jurkat cell were grown in medium, RPMI supplemented with 10% fetal bovine serum, 100U/ml penicillin and 100µg/ml streptomycin, collected and their suspension homogenized. After centrifugation, membrane fraction was separated, resuspended in phosphate buffer (pH=7.5) and stored in small aliquots in liquid nitrogen until use.
Binding of different radiolabeled ligans to Jurkat cell membranes was measured as described previously (Ramamoorthy et al., 1995). To characterize the σ binding sites in the Jurkat cell line, [³H]haloperidol as first used as the ligand. Haloperidol is a high affinity ligand to both type 1 and type 2 σ-receptors. The binding assays were done using Jurkat cell membranes in the presence of [³H]haloperidol (10nM) alone to determine the total binding, and in the presence of [³H]haloperidol (10nM) and unlabeled haloperidol (10µM) to determine the nonspecific binding.
Membranes were incubated with ligands in phosphate buffer for 3 hours at room temperature. After filter had been washed, radioactivity associated with the filter was determined by liquid scintillation spectrometry.

Compounds showing IC₅₀ and Kᵢ in concentrations lower than 1 µM, were considered to be active.

It is anticipated that similar results will be observed for other compounds of the invention.

### Forced swim test in mice

Male CD1 mice of the weight of 20-25 g were used for the experiment. Groups of 10 animals were treated with the test compounds, imipramine (positive control) or the vehicle (negative control) by *per os* by gavage 30 min prior to testing to determine efficacy. On the day of the experiment the animals were placed into a glass cylinder (height 18.2 cm, diameter 13.3 cm) filled with water warmed to 22 °C to the height of 10 cm. The immobility defined as the end of the struggling of the animal and the beginning of floating, wherein the movements were reduced to those indispensable for the animal to keep its head over the water surface, started to be recorded after two minutes and then it was monitored during 4 minutes.

The percentage of animals showing a passive behaviour was calculated and compared with a control group treated with a carrier. The compounds that in a dose of 10 mg/kg reduced the immobility of animals for 30 % and more over the control group were considered to be active.
It is anticipated that similar results will be observed for other compounds of the invention.

### Tail suspension test in mice

Male Balb/cJ mice of the weight of 20-25 g were used for the experiment. Groups of 9 animals were treated with the test compounds, imipramine (positive control) or the vehicle (negative control) by intraperitoneal injection, subcutaneous injection or per oral by gavage 30 min prior to testing to measure potential antidepressant activity. Mice were suspended from their tails at a height of about 90 cm and were observed for 5 minutes. The mice hanging fully motionless for 1 minute during the observation period were defined as depressive. In animals treated with a substance having an antidepressive action the period of immobility was shortened.

The percentage of animals showing a passive behaviour was calculated and compared with a control group treated with a vehicle. Significance of results was analysed using Fischer's exact test. The compounds that in a dose of 10 mg/kg reduced the immobility of animals for 40 % and more over a control group were considered to be active.
It is anticipated that similar results will be observed for other compounds of the invention.

### Amphetamine-induced hyperlocomotion in mice

Male Swiss OFA mice of a weight 30-35g were treated with either vehicle (saline) or test compounds 30 minutes prior to hyperlocomotion induction. Dexamphetamine sulphate was administered intraperitoneally at 2mg/kg. Thirty minutes later, animals were placed in a wooden box 80 x80 cm in a room with low light intensity (100 lux) for locomotor activity recording. Locomotor activity was determined during a 30 min period using a video image analyzer. Total duration of movement, occurence of movement and total distance travelled were measured. Haloperidol was tested at the dose of 0,25 mg/kg (prepared in 0,5% methylcelluloseand served as reference substance.

Compounds were considered as active if in a dose of 10 mg/kg reduced amphethamine-induced hyperlocomotion in experimental animals for 30% and more when compared to vehicle treated control group.
It is anticipated that similar results will be observed for other compounds of the invention.

### Meta-chlorophenyl piperazine (m-CPP) test on rats

The tested substance was administered to rats *per os* 1 hour before the test and m-CPP in a dose of 1 mg/kg was administered intravenously 15 minutes before the test. At the beginning of the experiment the treated animals were subjected to an open field test on rats (Drug Dev. Res. 1989, 18, 119-144): the apparatus consisted of an open box having the dimensions 80 × 65 × 35 cm, which in one wall had an opening with a diameter of 10 cm, by which it was connected to a non-illuminated compartment having the dimensions 25 × 21 × 21 cm, and the opening was illuminated by a light source (IR source or Kleverlux^{®}; 12 V/20 W) from the distance of 66 cm; one hour after administering the tested substance, the animals were placed in the dark (non-illuminated) compartment so that their heads were turned away from the illuminated exit and the passing of the animals from the dark compartment to the illuminated one was measured for 10 minutes.

As an active dose of the substance there was defined a dose at which the effect induced by m-CPP was reduced for 40 % and more.
It is anticipated that similar results will be observed for other compounds of the invention.

### Apomorphine, tryptamine, norepinephrine (ATN) test in rats

At the beginning of the experiment (t = 0) the animals were injected intravenously by 1.25 mg/kg of apomorphine, then by 40 mg/kg of tryptamine (t = 60 minutes) and by 1.25 mg/kg of norepinephrine (t = 90 minutes).

There were watched a state of exceptional agitation and normal behaviour during 60 minutes (apomorphine test), then bilateral clonic convulsions of back paws and a general tremor of the body in tryptamine test (observation period 5 minutes) and lethality during 120 minutes after the injection in norepinephrine test.

The percentage of animals showing a passive behaviour was calculated and compared with a control group treated with a carrier.

The compounds which in a dose of 10 mg/kg reduced the period of duration of observed effects (mobility) for 40 % over a control group were considered to be active in *in vivo* testings.
It is anticipated that similar results will be observed for other compounds of the invention.

Some of the present compounds tested in the above assays showed an action in at least two of said tests, though these results represent only an illustration of the biological action of the compounds.

### PREPARATION PROCESSES WITH EXAMPLES

The present invention is illustrated by the following Examples.

### Example 1

### 3-Methyl-3,3a-dihydro-2-oxa-8-thia-1-aza-dibenzo[e,h]azulen-3-ol (1a)

To a solution of 11H-dibenzo[*b,f*]thiepin-10-one oxime (1.66 mmole) in dry THF (10 mL) cooled to -78 °C, n-butyl lithium (3.57 mmole) was slowly added drop by drop. The reaction mixture was stirred for 15 minutes at this temperature, whereupon it was heated to 0 °C and ethyl acetate (3.57 mmole) was added thereto. The stirring of the reaction mixture was continued for 1 more hour at room temperature, whereupon water was added and it was extracted with ethyl acetate. The combined organic extracts were dried over anhydrous Na₂SO₄ and evaporated under reduced pressure. After purification by chromatography on a silica gel column, a crystalline product was isolated;
¹H NMR (ppm, CDCl₃): 2.03 (s, 3H), 7.27-7.60 (m, 8H);
MS (*m*/*z*): 306.1 [MNa⁺], 338.1 [MNa⁺ + MeOH].

### 3-Methyl-2-oxa-8-thia-1-aza-dibenzo[e,h]azulene (1)

To a solution of 3-methyl-3,3a-dihydro-2-oxa-8-thia-1-aza-dibenzo[*e,h*]azulen-3-ol **(1a)** (0.07 mmole) in THF (5 mL), concentrated sulfuric acid (100 µL) was added. The reaction mixture was stirred and heated under reflux for 5 hours, then it was cooled and the solvent was evaporated, water was added thereto and it was extracted with dichloromethane. The combined organic extracts were dried over anhydrous Na₂SO₄ and evaporated under reduced pressure. After purification by chromatography on a silica gel column, an oily product was isolated;
¹H NMR (ppm, CDCl₃): 2.74 (s, 3H), 7.35-7.93 (m, 8H);
MS (*m*/*z*): 265.9 [MH⁺].

### Example 2

### 3-Methyl-3,3a-dihydro-11-chloro-2-oxa-8-thia-1-aza-dibenzo[e,h]azulen-3-ol (2a)

To a solution of 11-chloro-11H-dibenzo[*b,f*]thiepin-10-one oxime (1.89 mmole) in dry THF (10 mL) cooled to -78 °C, n-butyl lithium (4.07 mmole) was slowly added drop by drop. The reaction mixture was stirred for 15 minutes at this temperature, whereupon it was heated to 0 °C and ethyl acetate (4.07 mmole) was added thereto. The stirring of the reaction mixture was continued for 1 more hour at room temperature, whereupon water was added and it was extracted with ethyl acetate. The combined organic extracts were dried over anhydrous Na₂SO₄ and evaporated under reduced pressure. After purification by chromatography on a silica gel column, a crystalline product was isolated;
MS (*m*/*z*): 340.1 [MNa⁺].

### 3-Methyl-11-chloro-2-oxa-8-titia-1-aza-dibenzo[e,h]azulene (2)

To a solution of 3-methyl-3,3a-dihydro-11-chloro-2-oxa-8-thia-1-aza-dibenzo[*e,h*]azulen-3-ol (2a) (0.08 mmole) in THF (5 mL), concentrated sulfuric acid (114 µL) was added. The reaction mixture was stirred and heated under reflux for 5 hours, then it was cooled and the solvent was evaporated, water was added thereto and it was extracted with dichloromethane. The combined organic extracts were dried over anhydrous Na₂SO₄ and evaporated under reduced pressure. After purification by chromatography on a silica gel column, an oily product was isolated;
MS (*m*/*z*): 300.78 [MH⁺].

### Example 3

### 3-Methyl-3,3a-dihydro-2,8-dioxa-1-aza-dibenzo[e,h]azulera-3-ol (3a)

To a solution of 11H-dibenzo[*b,f*]oxepin-10-one oxime (1.91 mmole) in dry THF (10 mL) cooled to -78 °C, n-butyl lithium (4.10 mmole) was slowly added drop by drop. The reaction mixture was stirred for 15 minutes at this temperature, whereupon it was heated to 0 °C and ethyl acetate (4.10 mmole) was added. The stirring of the reaction mixture was continued for one more hour at room temperature, whereupon water was added thereto and it was extracted with ethyl acetate. The combined organic extracts were dried over anhydrous Na₂SO₄ and evaporated under reduced pressure. After purification by chromatography on a silica gel column, a crystalline product was isolated;
MS (*m*/*z*): 290.3 [MNa⁺].

### 3-Methyl-2,8-dioxa-1-aza-dibenzo[e,h]azulene (3)

To a solution of 3-methyl-3,3a-dihydro-2,8-dioxa-1-aza-dibenzo[*e,h*]azulen-3-ol **(3a)** (0.1 mmole) in THF (7 mL), concentrated sulfuric acid (143 µL) was added. The reaction mixture was stirred and heated under the reflux for 5 hours, then it was cooled and the solvent was evaporated, water was added thereto and it was extracted with dichloromethane. The combined organic extracts were dried over anhydrous Na₂SO₄ and evaporated under reduced pressure. After purification by chromatography on a silica gel column, an oily product was isolated;
MS (*m*/*z*): 250.27 [MH⁺].

### Example 4

### 1-Bromomethyl-2-oxa-8-thia-1-aza-dibenzo[e,h]azulene (4)

To a solution of 3-methyl-2-oxa-8-thia-1-aza-dibenzo[*e,h*]azulene **(1)** (0.68 mmole) in carbon tetrachloride (15 mL), NBS (N-bromosuccinimide) (1.02 mmole) and a catalytic amount of benzoyl peroxide (PhCO)₂O₂ were added. The reaction mixture was stirred and heated under the reflux for 6-8 hours, then it was cooled, the precipitated succinimide was filtered and the solvent was evaporated, water was added thereto and it was extracted with dichloromethane. The combined organic extracts were dried over anhydrous Na₂SO₄ and evaporated under reduced pressure. After purification by chromatography on a silica gel column, an oily product was isolated;
¹H NMR (ppm, CDCl₃): 4.63 (s, 2H), 7.38-8.10 (m, 8H);
MS (*m*/*z*): 264.0 [M-Br].

### Example 5

### 1-Bromomethyl-11-chloro-2-oxa-8-thia-1-aza-dibenzo[e,h]azulene (5)

To a solution of 3-methyl-11-chloro-2-oxa-8-thia-1-aza-dibenzo[*e,h*]azulene (2) (0.78 mmole) in carbon tetrachloride (15 mL), NBS (N-bromosuccinimide) (1.17 mmole) and a catalytic amount of benzoyl peroxide (PhCO)₂O₂ were added. The reaction mixture was stirred and heated under reflux for 6-8 hours, then it was cooled, the precipitated succinimide was filtered and the solvent was evaporated, water was added thereto and it was extracted with dichloromethane. The combined organic extracts were dried over anhydrous Na₂SO₄ and evaporated under reduced pressure. After purification by chromatography on a silica gel column, a crystalline product was isolated;
MS (*m*/*z*): 298.45 [M-Br].

### Example 6

### 1-bromomethyl-2,8-dioxa-1-aza-dibenzo[e,h]azulene (6)

To a solution of 3-methyl-2,8-dioxa-1-aza-dibenzo[*e,h*]azulene **(3)** (0.58 mmole) in carbon tetrachloride (15 mL), NBS (N-bromosuccinimide) (0.87 mmole) and a catalytic amount of benzoyl peroxide (PhCO)₂O₂ were added. The reaction mixture was stirred and heated under reflux for 6-8 hours and cooled, the precipitated succinimide was filtered and the solvent was evaporated, water was added thereto and it was extracted with dichloromethane. The combined organic extracts were dried over anhydrous Na₂SO₄ and evaporated under reduced pressure. After purification by chromatography on a silica gel column, a crystalline product was isolated;
MS (*m*/*z*): 248.0 [M-Br].

### Example 7

### Dimethyl-[3-(2-oxa-8-thia-1-aza-dibenzo[e,h]azulen-3-ylmethoxy)-propyl]-amine (7)

To a solution of 3-dimethylaminopropylchloride-hydrochloride (2.16 mmole) in 50 % sodium hydroxide (1.9 mL), a catalytic amount of benzyltriethylammonium chloride and a solution of 1-bromomethyl-2-oxa-8-thia-1-aza-dibenzo[*e,h*]azulene **(4)** (0.15 mmole) in toluene (10 mL) were added. The reaction mixture was heated under vigorous stirring and reflux for 3 hours, then it was cooled to room temperature, diluted with water and extracted with dichloromethane. The organic extract was washed with water, dried over anhydrous Na₂SO₄ and evaporated under reduced pressure. After purification by chromatography on a silica gel column, a crystalline product was isolated;
MS (*m*/*z*): 367.2 [MH⁺].

### Example 8

### Dimethyl-[2-(2-oxa-8-thia-1-aza-dibenzo[e,h]azulen-3-ylmethoxy)-ethyl]-amine (8)

According to the process described in Example 7, starting from 1-bromomethyl-2-oxa-8-thia-1-aza-dibenzo[*e,h*]azulene **(4)** (0.20 mmole) and 2-dimethylaminoethylchloride-hydrochloride (2.85 mmole), an oily product was obtained;
¹H NMR (ppm, CDCl₃): 2.39 (s, 6H), 2.69-2.72 (t, 2H), 3.83-3.87 (t, 2H), 4.79 (s, 2H), 7.35-7.89 (m, 8H);
MS (*m*/*z*): 353.2 [MH⁺], 375.2 [MNa⁺].

### Example 9

### Dimethyl-[2-(11-chloro-2-oxa-8-thia-1-aza-dibenzo[e,h]azulen-3-ylmethoxy)-ethyl]-amine (9)

According to the process described in Example 7, starting from 1-bromomethyl-11-chloro-2-oxa-8-thia-1-aza-dibenzo[*e,h*]azulene **(5)** (0.18 mmole) and 2-dimethylaminoethylchloride-hydrochloride (2.56 mmole), an oily product was obtained;
MS (*m*/*z*): 387.65 [MH⁺].

### Example 10

### Dimethyl-[3-(11-chloro-2-oxa-8-thia-1-aza-dibenzo[e,h]azulen-3-ylmethoxy)-propyl]-amine (10)

According to the process described in Example 7, starting from 1-bromomethyl-11-chloro-2-oxa-8-thia-1-aza-dibenzo[*e,h*]azulene **(5)** (0.18 mmole) and 2-dimethylaminopropylchloride-hydrochloride (2.56 mmole), an oily product was obtained;
MS (*m*/*z*): 401.65 [MH⁺].

### Example 11

### Dimethyl-[2-(2,8-dioxa-1-aza-dibenzo[e,h]azulen-3-ylmethoxy)-ethyl]-amine (11)

According to the process described in Example 7, starting from 1-bromomethyl-2,8-dioxa-1-aza-dibenzo[*e,h*]azulene **(6)** (0.25 mmole) and 2-dimethylaminoethylchloride-hydrochloride (3.42 mmole), an oily product was obtained;
MS (*m*/*z*): 337.2 [MH⁺].

### Example 12

### Dimethyl-[3-(2,8-dioxa-1-aza-dibenzo[e,h]azulen-3-ylmethoxy)-propyl]-amine (12)

According to the process described in Example 7, starting from 1-bromomethyl-2,8-dioxa-1-aza-dibenzo[*e,h*]azulene **(6)** (0.25 mmole) and 2-dimethylaminopropylchloride-hydrochloride (3.42 mmole), an oily product was obtained;
MS (*m*/*z*): 351.2 [MH⁺].

### PREPARATION OF STARTING COMPOUNDS

### 11H-dibenzo[b,f]thiepin-10-one oxime

11H-dibenzo[*b,f*]thiepin-10-one (J.O. Jilek et al. Mh. Chem. 96 (1965) 182-207) (2.21 mmole) was dissolved in absolute ethanol (4.26 mL) and water (1.28 mL) under stirring and gentle heating. To the solution of ketone, aminehydroxide hydrochloride (4.42 mmole) and sodium acetate (4.42 mmole) were added. The reaction mixture was stirred and heated under reflux for 2 hours. After the completion of the reaction, 30 % ethanol (2 mL) was added into the hot reaction mixture and it was left to cool to room temperature. If no precipitation occurred, the solvent was evaporated under reduced pressure and the residue after evaporation was dissolved in water, extracted with dichloromethane, dried over anhydrous Na₂SO₄ and evaporated under reduced pressure. After purification by chromatography on a silica gel column, a crystalline product was isolated;
¹H NMR (ppm, CDCl₃): 3.65 (bs, 1H), 4.34 (s, 2H), 7.18-8.06 (m, 8H);
MS (*m*/*z*): 242.0 [MH⁺], 264.0 [MNa⁺], 296.0 [MNa⁺ + MeOH].

### 8-chloro-11H-dibenzo[b,f]thiepin-10-one oxime

11-chloro-11H-dibenzo[*b,f*]thiepin-10-one (J.O. Jilek et al. Mh. Chem. 96 (1965) 182-207) (1,47 mmole) was dissolved in absolute ethanol (2.84 mL) and water (0.9 mL) under stirring and gentle heating. To the solution of ketone, aminehydroxide hydrochloride (2.95 mmole) and sodium acetate (2.95 mmole) were added. The reaction mixture was stirred and heated under reflux for 2 hours. After the completion of the reaction, 30 % ethanol (1 mL) was added into the hot reaction mixture and it was left to cool to room temperature. If no precipitation occurred, the solvent was evaporated under reduced pressure and the residue after evaporation was dissolved in water, extracted with dichloromethane, dried over anhydrous Na₂SO₄ and evaporated under reduced pressure. After purification by chromatography on a silica gel column, a crystalline product was isolated;
MS (*m*/*z*): 276.45 [MH⁺].

### 11H-Dibenzo[b,f]oxepin-10-one oxime

11H-dibenzo[*b,f*]oxepin-10-one (I. Ueda et al. Chem. Pharm. Bull. 23 (10) 2223-2231 (1975)) (4.42 mmole) was dissolved in absolute ethanol (8.52 mL) and water (2.56 mL) under stirring and gentle heating. To the solution of ketone, aminehydroxide hydrochloride (8.84 mmole) and sodium acetate (8.84 mmole) were added. The reaction mixture was stirred and heated under reflux for 2 hours. After the completion of the reaction, 30 % ethanol (4 mL) was added into the hot reaction mixture and it was left to cool to room temperature. If no precipitation occurred, the solvent was evaporated under reduced pressure and the residue after evaporation was dissolved in water, extracted with dichloromethane, dried over anhydrous Na₂SO₄ and evaporated under reduced pressure. After purification by chromatography on a silica gel column, a crystalline product was isolated;
MS (*m*/*z*): 226.0 [MH⁺].

**Table 1**

| Compound | Structure | Name |
|---|---|---|
| **1a** | 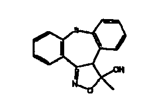 | 3-Methyl-3,3a-dihydro-2-oxa-8-thia-1-aza-dibenzo[*e,h*]azulen-3-ol |
| **2a** | 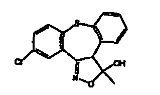 | 11-Chloro-3-methyl-3,3a-dihydro-2-axa-8-thia-1-aza-dibenzo[*e,h*]azulen-3-ol |
| **3a** | 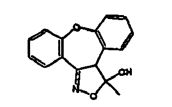 | 3-Methyl-3,3a-dihydro-2,8-dioxa-1-aza-dibenzo[*e,h*]azulen-3-ol |
| **1** | 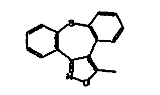 | 3-Methyl-2-oxo-8-thia-1-aza-dibenzo[*e,h*]azulene |
| **2** | 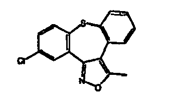 | 11-Chloro-3-methyl-2-oxa-8-thin-1-azn-dibenzo[*e,h*]azuiene |
| **3** | 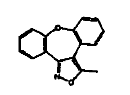 | 3-Methyl-2,8-dioxa-1-aza-dibenzo[*e,h*]azulene |
| **4** | 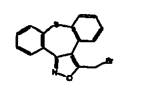 | 3-Bromomethyl-2-oxa-8-thin-1-azu-dibenzo[*e,h*]azulene |
| **5** | 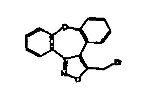 | 3-Bromomethyl-2,8-dioxa-1-aza-dibenzo[*e,h*]azulene |
| **6** | 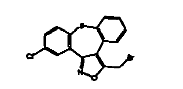 | 3-Bromomethyl-11-chloro-2-oxa-8-thin-1-aza-dibenzn[*e,h*]azulene |
| **7** | 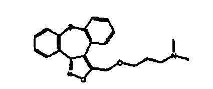 | Dimethyl-[3-(2-oxa-8-thin-1-aza-dibenzo[*e,h*]azulen-3-ylmethoxy)-propyl]amine |
| **8** | 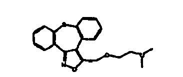 | Dimethyl-[2-(2-oxo-8-thia-1-azo-dibenzo[*e,h*]azulen-3-ylmethoxy)-ethyl]-amine |
| **9** | 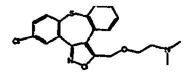 | [2-(11-Chloro-2-oxo-8-thin-1-aza-dibenzo[*e,h*]azulen-3-ylmethoxy)-ethyl]-dimethyl-amine |
| **10** | 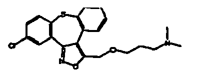 | [3-(11-Chloro-2-oxa-8-thin-1-aza-dibenzo[*e,h*]azulen-3-ylmethoxy)-propyl]-dimethyl-amine |
| **11** | 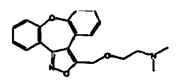 | [2-(2,8-Dioxa-1-azu-dibenzo[*e,h*]azulen-3-ylmethoxy)-ethyl]-dimethyl-amine |
| **12** | 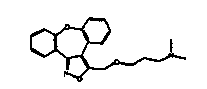 | [3-(2,8-Dioxo-1-azo-dibenzo[*e,h*]azulen-3-ylmethoxy)-propyl]-dimethyl-amine |

## Claims

1. A compound of formula **I:** 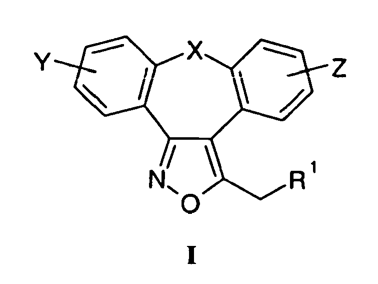 wherein
X means CH₂ or a heteroatom selected from the group consisting of O, S, S(=O), S(=O)₂ and NR^{a}, wherein R^{a} is hydrogen or a substituent selected from the group consisting of C₁-C₃-alkyl, C₁-C₃-alkanoyl, C₁-C₇-alkoxycarbonyl, C₇-C₁₀-arylalkyloxycarbonyl, C₇-C₁₀-aroyl, C₇-C₁₀-arylalkyl, C₃-C₇-alkylsilyl and C₅-C₁₀-alkylsilylalkyloxyalkyl;
Y and Z independently from each other mean one or more identical or different substituents linked to any available carbon atom selected from the group consisting of hydrogen, halogen, C₁-C₄-alkyl, C₂-C₄-alkenyl, C₂-C₄-alkinyl, halo-C₁-C₄-alkyl, hydroxy, C₁-C₄-alkoxy, trifluoromethoxy, C₁-C₄-alkanoyl, amino, amino-C₁-C₄-alkyl, C₁-C₄-alkylamino, *N*-(C₁-C₄-alkyl)amino, *N,N-*di(C₁-C₄-alkyl)amino, thiol, C₁-C₄-alkylthio, sulfonyl, C₁-C₄-alkylsulfonyl, sulfinyl, C₁-C₄-alkylsulfinyl, carboxy, C₁-C₄-alkoxycarbonyl, cyano and nitro;
R¹ means hydrogen, halogen, C₁-C₇-alkyl optionally substituted with one, two, three or more substituents selected from the group consisting of halogen atom, hydroxy, C₁-C₄ alkoxy, thiol, C₁-C₄ alkylthio, amino, N-(C₁-C₄) alkylamino, N,N-di(C₁-C₄-alkyl)-amino, sulfonyl, C₁-C₄ alkylsulfonyl, sulfinyl and C₁-C₄ alkylsulfinyl; C₂-C₇-alkenyl optionally substituted with one, two, three or more halogen atoms; C₂-C₇-alkinyl; monocyclic or bicyclic aryl group having from 6 to 10 carbon atoms and alternating double bonds and said group can be optionally substituted with one or two substituents selected from the group consisting of fluoro, chloro, C₁-C₄ alkyl, cyano, nitro, hydroxy, C₁-C₄ alkoxy, thiol, C₁-C₄ alkylthio, amino, *N*-(C₁-C₄) alkylamino, *N,N*-di(C₁-C₄-alkyl)amino, sulfonyl, C₁-C₄ alkylsulfonyl, sulfinyl, C₁-C₄ alkylsulfinyl and can be linked to the rest of the molecule by any available carbon atom via direct bond or via C₁-C₄ alkylene group; monocyclic or bicyclic heteroaryl having the meaning of aromatic and partially aromatic groups of a monocyclic or bicyclic ring with 4 to 12 carbon atoms and at least one of them being heteroatom selected from the group consisting of O, S and N wherein available carbon or nitrogen represent the binding site of the group to the rest of the molecule either via direct bond or via C₁-C₄ alkylene group and where said heteroaryl can be optionally substituted with fluoro, chloro, C₁-C₄ alkyl, cyano, nitro, hydroxy, C₁-C₄ alkoxy, thiol, C₁-C₄ alkylthio, amino, *N*-(C₁-C₄) alkylamino, *N*,*N*-di(C₁-C₄-alkyl)-amino, sulfonyl, C₁-C₄ alkylsulfonyl, sulfinyl, C₁-C₄ alkylsulfinyl; five-membered or six-membered fully saturated or partly unsaturated heterocycle group containing at least one hetero atom selected from the group consisting of O, S and N wherein available carbon or nitrogen represent the binding site of the group to the rest of the molecule either via direct bond or via C₁-C₄ alkylene group and where said heterocycle can be optionally substituted with fluoro, chloro, C₁-C₄ alkyl, cyano, nitro, hydroxy, C₁-C₄ alkoxy, thiol, C₁-C₄ alkylthio, amino, *N*-(C₁-C₄) alkylamino, *N*,*N*-di(C₁-C₄-alkyl)-amino, sulfonyl, C₁-C₄ alkylsulfonyl, sulfinyl, C₁-C₄ alkylsulfinyl; hydroxy; hydroxy-C₂-C₇-alkenyl; hydroxy-C₂-C₇-alkinyl; C₁-C₇-alkoxy; thiol; thio-C₂-C₇-alkenyl; thio-C₂-C₇-alkinyl; C₁-C₇-alkylthio; amino; *N*-(C₁-C₇-alkyl)amino; *N*,*N*-di(C₁-C₇-alkyl)amino; C₁-C₇-alkylamino; amino-C₂-C₇-alkenyl; amino-C₂-C₇-alkinyl; amino-C₁-C₇-alkoxy; C₁-C₇-alkanoyl; C₇-C₁₀-aroyl; oxo-C₁-C₇-alkyl; C₁-C₇-alkanoyloxy; carboxy; C₁-C₇-alkyloxycarbonyl, which is optionally substituted with one, two, three or more substituents selected from the group consisting of halogen, hydroxy, C₁-C₄-alkoxy, thiol, C₁-C₄-alkylthio, amino, N-(C₁-C₄-alkyl)amino, N,N-di(C₁-C₄-alkyl)amino, sulfonyl, C₁-C₄-alkylsulfonyl, sulfinyl, and C₁-C₄-alkylsulfinyl; C₇-C₁₀-aryloxycarbonyl, which is optionally substituted with one or two substituents selected from the group consisting of halogen, C₁-C₄-alkyl, cyano, nitro, hydroxy, C₁-C₄-alkoxy, thiol, C₁-C₄-alkylthio, amino, N-(C₁-C₄)alkylamino, N,N-di(C₁-C₄-alkyl)amino, sulfonyl, C₁-C₄-alkylsulfonyl, sulfinyl, C₁-C₄-alkylsulfinyl; *N*-(C₁-C₇-alkyl)carbamoyl; *N*,*N*-di(C₁-C₇-alkyl)carbamoyl; cyano; cyano-C₁-C₇-alkyl; sulfonyl; C₁-C₇-alkylsulfonyl; sulfinyl; C₁-C₇-alkylsulfinyl; nitro;
or a substituent represented with the formula **II:** 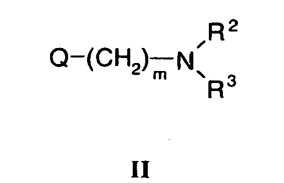
wherein
R² and R³ simultaneously or independently from each other have the meaning of hydrogen, C₁-C₄-alkyl, aryl having the meaning as defined above or together with N have the meaning of optionally substituted heterocycle or heteroaryl wherein heterocycle relates to five-membered or six-membered fully saturated or partly unsaturated heterocycle group containing at least one hetero atom selected from the group consisting of O, S and N and where said heterocycle can be optionally substituted with one or two substituents which are selected from halogen, C₁-C₄ alkyl, cyano, nitro, hydroxy, C₁-C₄ alkoxy, thiol, C₁-C₄ alkylthio, amino, *N*-(C₁-C₄) alkylamino, *N*,*N*-di(C₁-C₄-alkyl)-amino, sulfonyl, C₁-C₄ alkylsulfonyl, sulfinyl, C₁-C₄ alkylsulfinyl and heteroaryl relates to aromatic and partially aromatic groups of a monocyclic or bicyclic ring with 4 to 12 carbon atoms and at least one of them being heteroatom selected from the group consisting of O, S and N and where said heteroaryl can be optionally substituted with one or two substituents which are selected from halogen, C₁-C₄ alkyl, cyano, nitro, hydroxy, C₁-C₄ alkoxy, thiol, C₁-C₄ alkylthio, amino, *N*-(C₁-C₄) alkylamino , *N*,*N-*di(C₁-C₄-alkyl)-amino, sulfonyl; C₁-C₄ alkylsulfonyl, sulfinyl, C₁-C₄ alkylsulfinyl;
m has the meaning of an integer from 1 to 3;
Q has the meaning of oxygen, sulfur or NH;
and pharmaceutically acceptable salts and solvates thereof.

2. A compound according to claim 1 wherein X represents O or S.

3. A compound according to claim 1 wherein Y and Z independently from each other mean one or more identical or different substituents linked to any available carbon atom selected from the group consisting of hydrogen, fluorine, chlorine, bromine, C₁-C₄-alkyl, halo-C₁-C₄-alkyl, hydroxy, C₁-C₄-alkoxy, trifluoromethoxy, C₁-C₄-alkanoyl, amino, amino-C₁-C₄-alkyl, *N*-(C₁-C₄-alkyl)amino, *N*,*N*-di(C₁-C₄-alkyl)amino, thiol, C₁-C₄-alkylthio, cyano and nitro.

4. A compound according to claim 1 wherein R¹ has the maning of hydrogen, halogen, C₁-C₇-alkyl optionally substituted with one, two, three or more substituents selected from the group consisting of halogen atom, hydroxy, C₁-C₄ alkoxy, thiol, C₁-C₄ alkylthio, amino, *N*-(C₁-C₄) alkylamino and *N,N*-di(C₁-C₄-alkyl)-amino; monocyclic or bicyclic aryl group having from 6 to 10 carbon atoms and alternating double bonds and said group can be optionally substituted with one or two substituents selected from the group consisting of fluoro, chloro, C₁-C₄ alkyl, cyano, nitro, hydroxy, C₁-C₄ alkoxy, thiol, C₁-C₄ alkylthio, amino, *N*-(C₁-C₄) alkylamino and *N*,*N*-di(C₁-C₄-alkyl)-amino and can be linked to the rest of the molecule by any available carbon atom via direct bond or via C₁-C₄ alkylene group; monocyclic or bicyclic heteroaryl having the meaning of aromatic and partially aromatic groups of a monocyclic or bicyclic ring with 4 to 12 carbon atoms and at least one of them being heteroatom selected from the group consisting of O, S and N wherein available carbon or nitrogen represent the binding site of the group to the rest of the molecule either via direct bond or via C₁-C₄ alkylene group and where said heteroaryl can be optionally substituted with fluoro, chloro, C₁-C₄ alkyl, cyano, nitro, hydroxy, C₁-C₄ alkoxy, thiol, C₁-C₄ alkylthio, amino, *N*-(C₁-C₄) alkylamino and *N,N*-di(C₁-C₄-alkyl)-amino; five-membered or six-membered fully saturated or partly unsaturated heterocycle group containing at least one hetero atom selected from the group consisting of O, S and N wherein available carbon or nitrogen represent the binding site of the group to the rest of the molecule either via direct bond or via C₁-C₄ alkylene group and where said heterocycle can be optionally substituted with fluoro, chloro, C₁-C₄ alkyl, cyano, nitro, hydroxy, C₁-C₄ alkoxy, thiol, C₁-C₄ alkylthio, amino, *N*-(C₁-C₄) alkylamino and *N*,*N*-di(C₁-C₄-alkyl)-amino; hydroxy; C₁-C₇-alkoxy; thiol; C₁-C₇-alkylthio; amino; *N-*(C₁-C₇-alkyl)amino; *N*,*N*-di(C₁-C₇-alkyl)amino; amino-C₁-C₇-alkoxy; C₁-C₇-alkanoyl; C₇-C₁₀-aroyl; C₁-C₇-alkanoyloxy; an C₁-C₇-alkyloxycarbonyl, which is optionally substituted with one, two, three or more substituents selected from the group consisting of halogen, hydroxy, C₁-C₄-alkoxy, thiol, C₁-C₄-alkylthio, amino, N-(C₁-C₄-alkyl)amino, N,N-di(C₁-C₄-alkyl)amino, sulfonyl, C₁-C₄-alkylsulfonyl, sulfinyl, and C₁-C₄-alkylsulfinyl; C₇-C₁₀-aryloxycarbonyl, which is optionally substituted with one or two substituents selected from the group consisting of halogen, C₁-C₄-alkyl, cyano, nitro, hydroxy, C₁-C₄-alkoxy, thiol, C₁-C₄-alkylthio, amino, N-(C₁-C₄)alkylamino, N,N-di(C₁-C₄-alkyl)amino, sulfonyl, C₁-C₄-alkylsulfonyl, sulfinyl, C₁-C₄-alkylsulfinyl, carbamoyl; *N*-(C₁-C₇-alkyl)carbamoyl; *N*,*N*-di(C₁-C₇-alkyl)carbamoyl; cyano; cyano-C₁-C₇-alkyl; nitro;
or a substituent represented with the formula **II**: 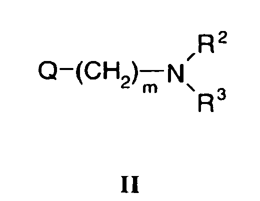 wherein
R² and R³ simultaneously or independently from each other have the meaning of hydrogen, C₁-C₄-alkyl, aryl having the meaning as described above; or together with N have the meaning of heterocycle or heteroaryl selected from the group consisting of morpholine-4-yl, piperidine-1-yl, pyrrolidine-1-yl, imidazole-1-yl and piperazine-1-yl;
m has the meaning of an integer from 1 to 3;
Q has the meaning of oxygen.

5. A compound according to claims 1 or 3 wherein Y represents hydrogen or chlorine and Z represents hydrogen.

6. A compound according to claims 1 or 4 wherein R¹ represents CH₃, CH₂Br, CH₂OH or a substituent of formula **II:** 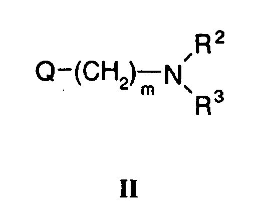 wherein R², R³, Q and m have the above defined meaning.

7. A compound according to claim 6 wherein the symbol m has the meaning of 2 or 3.

8. A compound according to claim 1 selected from the group consisting of:
3-methyl-2-oxa-8-thia-1-aza-dibenzo[*e,h*]azulene;
11-chloro-3-methyl-2-oxa-8-thia-1-aza-dibenzo[*e,h*]azulene;
3-methyl-2,8-dioxa-1-aza-dibenzo[*e,h*]azulene;
3-bromomethyl-2-oxa-8-thia-1-aza-dibenzo[*e,h*]azulene;
3-bromomethyl-11-chloro-2-oxa-8-thia-1-aza-dibenzo[*e*,*h*]azulene;
3-bromomethyl-2,8-dioxa-1-aza-dibenzo[*e,h*]azulene;
dimethyl-[2-(2-oxa-8-thia-1-aza-dibenzo[*e,h*]azulen-3-ylmethoxy)-ethyl]-amine;
dimethyl-[3-(2-oxa-8-thia-1-aza-dibenzo[*e,h*]azulen-3-ylmethoxy)-propyl]-amine;
dimethyl-[2-(11-chloro-2-oxa-8-thia-1-aza-dibenzo[*e,h*]azulen-3-ylmethoxy)-ethyl]-amine;
dimethyl-[3-(11-chloro-2-oxa-8-thia-1-aza-dibenzo[*e,h*]azulen-3-ylmethoxy)-propyl]-amine;
dimethyl-[2-(2,8-dioxa-1-aza-dibenzo[*e*,*h*]azulen-3-ylmethoxy)-ethyl]-amine; and
dimethyl-[3-(2,8-dioxa-1-aza-dibenzo[*e*,*h*]azulen-3-ylmethoxy)-propyl]-amine.

9. Process for the preparation of the compound of the formula **I**: 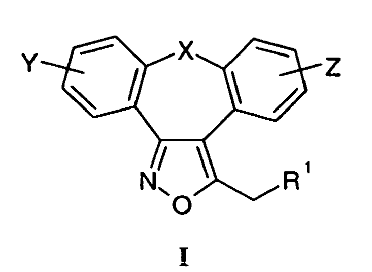 wherein
X means CH₂ or a heteroatom selected from the group consisting of O, S, S(=O), S(=O)₂ and NR^{a}, wherein R^{a} is hydrogen or a substituent selected from the group consisting of C₁-C₃-alkyl, C₁-C₃-alkanoyl, C₁-C₇-alkoxycarbonyl, C₇-C₁₀-arylalkyloxycarbonyl, C₇-C₁₀-aroyl, C₇-C₁₀-arylalkyl, C₃-C₇-alkylsilyl and C₅-C₁₀-alkylsilylalkyloxyalkyl;
Y and Z independently from each other mean one or more identical or different substituents linked to any available carbon atom selected from the group consisting of hydrogen, halogen, C₁-C₄-alkyl, C₂-C₄-alkenyl, C₂-C₄-alkinyl, halo-C₁-C₄-alkyl, hydroxy, C₁-C₄-alkoxy, trifluoromethoxy, C₁-C₄-alkanoyl, amino, amino-C₁-C₄-alkyl, C₁-C₄-alkylamino, *N*-(C₁-C₄-alkyl)amino, *N,N-*di(C₁-C₄-alkyl)amino, thiol, C₁-C₄-alkylthio, sulfonyl, C₁-C₄-alkylsulfonyl, sulfinyl, C₁-C₄-alkylsulfinyl, carboxy, C₁-C₄-alkoxycarbonyl, cyano and nitro;
R¹ means hydrogen, halogen, C₁-C₇-alkyl optionally substituted with one, two, three or more substituents selected from the group consisting of halogen atom, hydroxy, C₁-C₄ alkoxy, thiol, C₁-C₄ alkylthio, amino, N-(C₁-C₄) alkylamino, N,N-di(C₁-C₄-alkyl)-amino, sulfonyl, C₁-C₄ alkylsulfonyl, sulfinyl and C₁-C₄ alkylsulfinyl; C₂-C₇-alkenyl optionally substituted with one, two, three or more halogen atoms; C₂-C₇-alkinyl; monocyclic or bicyclic aryl group having from 6 to 10 carbon atoms and alternating double bonds and said group can be optionally substituted with one or two substituents selected from the group consisting of fluoro, chloro, C₁-C₄ alkyl, cyano, nitro, hydroxy, C₁-C₄ alkoxy, thiol, C₁-C₄ alkylthio, amino, *N*-(C₁-C₄) alkylamino, *N,N*-di(C₁-C₄-alkyl)-amino, sulfonyl, C₁-C₄ alkylsulfonyl, sulfinyl, C₁-C₄ alkylsulfinyl and can be linked to the rest of the molecule by any available carbon atom via direct bond or via C₁-C₄ alkylene group; monocyclic or bicyclic heteroaryl having the meaning of aromatic and partially aromatic groups of a monocyclic or bicyclic ring with 4 to 12 carbon atoms and at least one of them being heteroatom selected from the group consisting of O, S and N wherein available carbon or nitrogen represent the binding site of the group to the rest of the molecule either via direct bond or via C₁-C₄ alkylene group and where said heteroaryl can be optionally substituted with fluoro, chloro, C₁-C₄ alkyl, cyano, nitro, hydroxy, C₁-C₄ alkoxy, thiol, C₁-C₄ alkylthio, amino, *N*-(C₁-C₄) alkylamino, *N,N*-di(C₁-C₄-alkyl)-amino, sulfonyl, C₁-C₄ alkylsulfonyl, sulfinyl, C₁-C₄ alkylsulfinyl; five-membered or six-membered fully saturated or partly unsaturated heterocycle group containing at least one hetero atom selected from the group consisting of O, S and N wherein available carbon or nitrogen represent the binding site of the group to the rest of the molecule either via direct bond or via C₁-C₄ alkylene group and where said heterocycle can be optionally substituted with fluoro, chloro, C₁-C₄ alkyl, cyano, nitro, hydroxy, C₁-C₄ alkoxy, thiol, C₁-C₄ alkylthio, amino, *N*-(C₁-C₄) alkylamino, *N,N*-di(C₁-C₄-alkyl)-amino, sulfonyl, C₁-C₄ alkylsulfonyl, sulfinyl, C₁-C₄ alkylsulfinyl; hydroxy; hydroxy-C₂-C₇-alkenyl; hydroxy-C₂-C₇-alkinyl; C₁-C₇-alkoxy; thiol; thio-C₂-C₇-alkenyl; thio-C₂-C₇-alkinyl; C₁-C₇-alkylthio; amino; *N*-(C₁-C₇-alkyl)amino; *N,N*-di(C₁-C₇-alkyl)amino; C₁-C₇-alkylamino; amino-C₂-C₇-alkenyl; amino-C₂-C₇-alkinyl; amino-C₁-C₇-alkoxy; C₁-C₇-alkanoyl; C₇-C₁₀-aroyl; oxo-C₁-C₇-alkyl; C₁-C₇-alkanoyloxy; carboxy; C₁-C₇-alkyloxycarbonyl, which is optionally substituted with one, two, three or more substituents selected from the group consisting of halogen, hydroxy, C₁-C₄-alkoxy, thiol, C₁-C₄-alkylthio, amino, N-(C₁-C₄-alkyl)amino, N,N-di(C₁-C₄-alkyl)amino, sulfonyl, C₁-C₄-alkylsulfonyl, sulfinyl, and C₁-C₄-alkylsulfinyl,; C₇-C₁₀-aryloxycarbonyl, which is optionally substituted with one or two substituents selected from the group consisting of halogen, C₁-C₄-alkyl, cyano, nitro, hydroxy, C₁-C₄-alkoxy, thiol, C₁-C₄-alkylthio, amino, N-(C₁-C₄)alkylamino, N,N-di(C₁-C₄-alkyl)amino, sulfonyl, C₁-C₄-alkylsulfonyl, sulfinyl, C₁-C₄-alkylsulfinyl,; *N*-(C₁-C₇-alkyl)carbamoyl; *N,N*-di(C₁-C₇-alkyl)carbamoyl; cyano; cyano-C₁-C₇-alkyl; sulfonyl; C₁-C₇-alkylsulfonyl; sulfinyl; C₁-C₇-alkylsulfinyl; nitro;
or a substituent represented with the formula **II:** 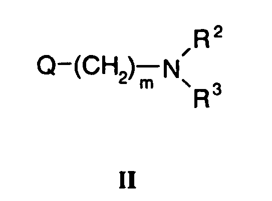
wherein
R² and R³ simultaneously or independently from each other have the meaning of hydrogen, C₁-C₄-alkyl, aryl having the meaning as defined above, or together with N have the meaning of optionally substituted heterocycle or heteroaryl wherein heterocycle relates to five-membered or six-membered fully saturated or partly unsaturated heterocycle group containing at least one hetero atom selected from the group consisting of O, S and N and where said heterocycle can be optionally substituted with one or two substituents which are selected from halogen, C₁-C₄ alkyl, cyano, nitro, hydroxy, C₁-C₄ alkoxy, thiol, C₁-C₄ alkylthio, amino, *N*-(C₁-C₄) alkylamino, *N,N*-di(C₁-C₄-alkyl)-amino, sulfonyl, C₁-C₄ alkylsulfonyl, sulfinyl, C₁-C₄ alkylsulfinyl; and wherein heteroaryl relates to aromatic and partially aromatic groups of a monocyclic or bicyclic ring with 4 to 12 carbon atoms and at least one of them being heteroatom selected from the group consisting of O, S and N and where said heteroaryl can be optionally substituted with one or two substituents which are selected from halogen, C₁-C₄ alkyl, cyano, nitro, hydroxy, C₁-C₄ alkoxy, thiol, C₁-C₄ alkylthio, amino, *N*-(C₁-C₄) alkylamino , *N,N*-di(C₁-C₄-alkyl)-amino, sulfonyl, C₁-C₄ alkylsulfonyl, sulfinyl, C₁-C₄ alkylsulfinyl;
m has the meaning of an integer from 1 to 3 and
Q has the meaning of oxygen, sulfur or NH;
and its pharmacologically acceptable salts and solvates, which comprises:
a) condensation of a compound **Ia:** 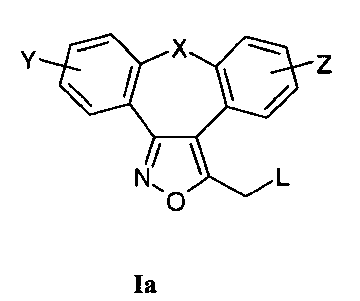 wherein symbols X, Y and Z have the meaning as defined above, L has the meaning of a leaving group,
with an optionally selected alcohol, thioalcohol or amine or with a compound of the formula **IIa:** 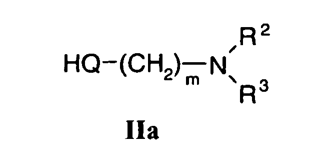 wherein all radicals and symbols have earlier stated meanings; or
b) condensation of a compound of the formula **Ib:** 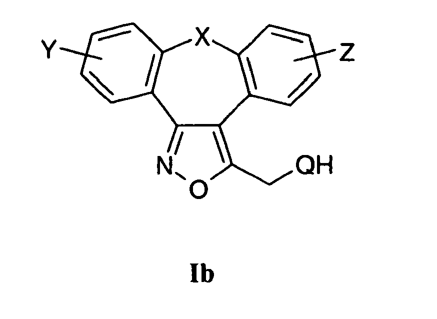
wherein all symbols have the earlier stated meanings, with a compound of the formula **IIb**: 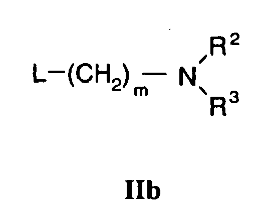 wherein the radicals R² and R³ and the symbol m have the earlier stated meanings and symbol L has the meaning of a suitable leaving group.

10. A pharmaceutical composition comprising at least one compound according to claim 1 and pharmaceutically acceptable salt or solvate thereof in association with a pharmaceutically acceptable excipient diluent and/or carrier.

11. Use of a compound according to claim 1 for the manufacture of a pharmaceutical formulation for the treatment and prevention of diseases, damages and disorders of the central nervous system caused by disorders of neurochemical equilibrium of biogenic amines or other neurotransmitters

12. Use according to claim 11, wherein the selected biogenic amines are serotonin, norepinephrine and dopamine.

13. Use according to claim 11, wherein neurotransmitter is glutamate.

14. Use according to claims 11, 12 or 13 wherein the compounds of the general formula I act upon the neurochemical equilibrium by regulating the synthesis, storing, releasing, metabolizing and/or reabsorption of biogenic amines or neurotransmitters and binding to their receptors.

15. Use according to claim 14, wherein the compounds of the general formula I show binding affinity to a receptor of one or more biogenic amines.

16. Use according to claim 15, wherein the compounds of the general formula I show a significant binding affinity to serotonin 5-HT_{2A} and 5-HT_{2C} receptors.

17. Use according to claim 16, wherein the compounds of the general formula I show binding affinity to selected serotonin receptors in a concentration of IC₅₀<1µM.

18. Use according to claim 11, wherein the compounds of the general formula I act as σ1 receptor ligands in a concentration of IC₅₀<1µM by modulating central neurotransmitter system.

19. Use according to claims 11, 16 or 18, wherein the compounds of the general formula I show dual binding affinity to σ1 receptor and to at least one serotonin receptor selected from 5-HT_{2A} and 5-HT_{2C}.

20. Use according to claim 11, wherein the diseases and disorders of the central nervous system are selected from the group consisting of anxiety, depression and modest depression, bipolar disorders, sleeping disorders, sexual disorders, psychosis, borderline psychosis, schizophrenia, migraine, personality disorders and obsessive-compulsive disorders, social phobia or panic attacks, organic mental disorders in children, aggression, memory disorders and personality disorders in elderly people, addiction, obesity, bulimia and similar disorders, snoring, premenstrual troubles.

21. Use according to claim 11, wherein the damages of the central nervous system are caused by trauma, brain stroke, neurodegenerative diseases, cardiovascular disorders such as high blood pressure, thrombosis, infarct as well as by gastrointestinal disorders.

22. Use according to claim 11, wherein the compounds of the general formula I, pharmaceutically acceptable salts and solvates thereof are selected from the group consisting of:
3-methyl-2-oxa-8-thia-1-aza-dibenzo[*e,h*]azulene;
11-chloro-3-methyl-2-oxa-8-thia-1-aza-dibenzo[*e,h*]azulene;
3-methyl-2,8-dioxa-1-aza-dibenzo[*e,h*]azulene;
3-bromomethyl-2-oxa-8-thia-1-aza-dibenzo[*e,h*]azulene;
3-bromomethyl-11-chloro-2-oxa-8-thia-1-aza-dibenzo[*e,h*]azulene;
3-bromomethyl-2,8-dioxa-1-aza-dibenzo[*e,h*]azulene;
dimethyl-[2-(2-oxa-8-thia-1-aza-dibenzo[*e,h*]azulen-3-ylmethoxy)-ethyl]-amine;
dimethyl-[3-(2-oxa-8-thia-1-aza-dibenzo[*e,h*]azulen-3-ylmethoxy)-propyl]-amine;
dimethyl-[2-(11-chloro-2-oxa-8-thia-1-aza-dibenzo[*e,h*]azulen-3-ylmethoxy)-ethyl]-amine;
dimethyl-[3-(11-chloro-2-oxa-8-thia-1-aza-dibenzo[*e,h*]azulen-3-ylmethoxy)-propyl]-amine;
dimethyl-[2-(2,8-dioxa-1-aza-dibenzo[*e,h*]azulen-3-ylmethoxy)-ethyl]-amine;and
dimethyl-[3-(2,8-dioxa-1-aza-dibenzo[*e,h*]azulen-3-ylmethoxy)-propyl]-amine.

## Patentansprüche

1. Verbindung der Formel **I**: 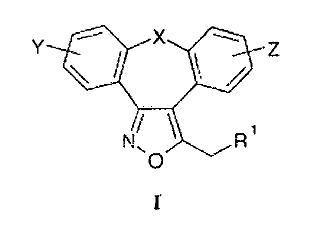 wobei
X für CH₂ oder ein Heteroatom steht, das aus der Gruppe bestehend aus O, S, S(=O), S(=O)₂ und NR^{a} ausgewählt ist, wobei R^{a} Wasserstoff oder ein Substituent ist, der aus der Gruppe bestehend aus C₁-C₃-Alkyl, C₁-C₃-Alkanoyl, C₁-C₇-Alkoxycarbonyl, C₇-C₁₀-Arylalkoxycarbonyl, C₇-C₁₀-Aroyl, C₇-C₁₀-Arylalkyl, C₃-C₇-Alkylsilyl und C₅-C₁₀-Alkylsilylalkyloxyalkyl ausgewählt ist;
Y und Z unabhängig voneinander für einen oder mehrere identische oder unterschiedliche Substituenten stehen, die an ein beliebiges verfügbares Kohlenstoffatom gebunden sind, und die aus der Gruppe bestehend aus Wasserstoff, Halogen, C₁-C₄-Alkyl, C₂-C₄-Alkenyl, C₂-C₄-Alkinyl, Halogen-C₁-C₄-alkyl, Hydroxy, C₁-C₄-Alkoxy, Trifluormethoxy, C₁-C₄-Alkanoyl, Amino, Amino-C₁-C₄-alkyl, C₁-C₄-Alkylamino, *N*-(C₁-C₄-Alkyl)amino, *N,N*-Di(C₁-C₄-alkyl)amino, Thiol, C₁-C₄-Alkylthio, Sulfonyl, C₁-C₄-Alkylsulfonyl, Sulfinyl, C₁-C₄-Alkylsulfinyl, Carboxy, C₁-C₄-Alkoxycarbonyl, Cyano und Nitro ausgewählt sind;
R¹ für Wasserstoff, Halogen, gegebenenfalls substituiertes C₁-C₇-Alkyl, das gegebenenfalls mit einem, zwei, drei oder mehr Substituenten, die aus der Gruppe bestehend aus Halogenatom, Hydroxy, C₁-C₄-Alkoxy, Thiol, C₁-C₄-Alkylthio, Amino, *N*-(C₁-C₄)alkylamino, *N,N*-Di(C₁-C₄-alkyl)-amino, Sulfonyl, C₁-C₄-Alkylsulfonyl, Sulfinyl und C₁-C₄-Alkylsulfinyl ausgewählt sind, substituiert ist, C₂-C₇-Alkenyl, welches gegebenenfalls mit einem, zwei, drei oder mehr Halogenatomen substituiert ist; C₂-C₇-Alkinyl; einen monocyclischen oder bicyclischen Arylrest, der 6 bis 10 Kohlenstoffatome und alternierende Doppelbindungen aufweist und der gegebenenfalls mit einem oder zwei Substituenten, die aus der Gruppe bestehend aus Fluor, Chlor, C₁-C₄-Alkyl, Cyano, Nitro, Hydroxy, C₁-C₄-Alkoxy, Thiol, C₁-C₄-Alkylthio, Amino, *N*-(C₁-C₄)Alkylamino, *N,N*-Di(C₁-C₄-alkyl)-amino, Sulfonyl, C₁-C₄-Alkylsulfonyl, Sulfinyl, C₁-C₄-Alkylsulfinyl ausgewählt sind, substituiert ist und mit einem beliebigen verfügbaren Kohlenstoffatom über direkte Bindung oder mit einem C₁-C₄-Alkylenrest an den Rest des Moleküls gebunden sein kann; monocyclisches oder bicyclisches Heteroaryl, das für aromatische oder partiell aromatische Reste eines monocyclischen oder bicyclischen Rings mit 4 bis 12 Kohlenstoffatomen steht und mindestens eines von ihnen ein Heteroatom ist, das aus der Gruppe bestehend aus O, S und N ausgewählt ist, wobei verfügbarer Kohlenstoff oder Stickstoff die Bindungsstelle des Restes zum Rest des Moleküls entweder über eine direkte Bindung oder einen C₁-C₄-Alkylenrest darstellt und wobei Heteroaryl gegebenenfalls mit Fluor, Chlor, C₁-C₄-Alkyl, Cyano, Nitro, Hydroxy, C₁-C₄-Alkoxy, Thiol, C₁-C₄-Alkylthio, Amino, *N*-(C₁-C₄)Alkylamino, *N,N*-Di(C₁-C₄-alkyl)-amino, Sulfonyl, C₁-C₄-Alkylsulfonyl, Sulfinyl, C₁-C₄-Alkylsulfinyl substituiert sein kann; einen fünfgliedrigen oder sechsgliedrigen völlig gesättigten oder teilweise ungesättigten heterocyclischen Rest, der mindestens ein Heteroatom, das aus der Gruppe bestehend aus O, S und N ausgewählt ist, enthält, wobei verfügbarer Kohlenstoff oder Stickstoff die Bindungsstelle des Rests an den Rest des Moleküls entweder über eine direkte Bindung oder über einen C₁-C₄-Alkylenrest darstellt und wobei der Heterocyclus gegebenenfalls mit Fluor, Chlor, C₁-C₄-Alkyl, Cyano, Nitro, Hydroxy, C₁-C₄-Alkoxy, Thiol, C₁-C₄-Alkylthio, Amino, *N*-(C₁-C₄)Alkylamino, *N,N*-Di(C₁-C₄-alkyl)-amino, Sulfonyl, C₁-C₄-Alkylsulfonyl, Sulfinyl, C₁-C₄-Alkylsulfinyl substituiert sein kann; Hydroxy; Hydroxy-C₂-C₇-alkenyl; Hydroxy-C₂-C₇-alkinyl; C₁-C₇-Alkoxy; Thiol; Thio-C₂-C₇-alkenyl; Thio-C₂-C₇-alkinyl; C₁-C₇-Alkylthio; Amino; *N*-(C₁-C₇-Alkyl)amino; *N,N*-Di(C₁-C₇-alkyl)amino; C₁-C₇-Alkylamino; Amino-C₂-C₇-alkenyl; Amino-C₂-C₇-alkinyl; Amino-C₁-C₇-alkoxy; C₁-C₇-Alkanoyl; C₇-C₁₀-Aroyl; Oxo-C₁-C₇-alkyl; C₁-C₇-Alkanoyloxy; Carboxy; C₁-C₇-Alkyloxycarbonyl, welches gegebenenfalls mit einem, zwei, drei oder mehr Substituenten substituiert ist, die aus der Gruppe bestehend aus Halogen, Hydroxy, C₁-C₄-Alkoxy, Thiol, C₁-C₄-Alkylthio, Amino, *N*-(C₁-C₄-Alkyl)amino, N,*N*-Di(C₁-C₄-alkyl)amino, Sulfonyl, C₁-C₄-Alkylsulfonyl, Sulfinyl und C₁-C₄-Alkylsulfinyl ausgewählt sind; C₇-C₁₀-Aryloxycarbonyl, welches gegebenenfalls substituiert ist mit einem oder zwei Substituenten, die aus der Gruppe bestehend aus Halogen, C₁-C₄-Alkyl, Cyano, Nitro, Hydroxy, C₁-C₄-Alkoxy, Thiol, C₁-C₄-Alkylthio, Amino, *N*-(C₁-C₄)Alkylamino, *N,N*-Di(C₁-C₄-alkyl)amino, Sulfonyl, C₁-C₄-Alkylsulfonyl, Sulfinyl, C₁-C₄-Alkylsulfinyl ausgewählt sind; *N*-(C₁-C₇-Alkyl)carbamoyl; *N,N-*Di(C₁-C₇-alkyl)carbamoyl; Cyano; Cyano-C₁-C₇-alkyl; Sulfonyl; C₁-C₇-Alkylsulfonyl; Sulfinyl; C₁-C₇-Alkylsulfinyl; Nitro steht;
oder für einen Substituenten, der durch Formel **II** dargestellt ist: 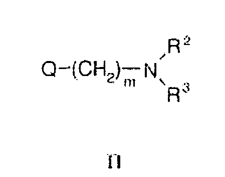
wobei
R² und R³ gleichzeitig oder unabhängig voneinander für Wasserstoff, C₁-C₄-Alkyl, Aryl, das die oben definierte Bedeutung hat, stehen, oder zusammen mit N für einen gegebenenfalls substituierten Heterocyclus oder Heteroaryl stehen, wobei sich der Heterocyclus auf einen fünfgliedrigen oder sechsgliedrigen völlig gesättigten oder teilweise ungesättigten heterocyclischen Rest bezieht, der mindestens ein Heteroatom enthält, das aus der Gruppe bestehend aus O, S und N ausgewählt ist und wobei der Heterocyclus gegebenenfalls mit einem oder zwei Substituenten substituiert sein kann, welche aus Halogen, C₁-C₄-Alkyl, Cyano, Nitro, Hydroxy, C₁-C₄-Alkoxy, Thiol, C₁-C₄-Alkylthio, Amino, *N*-(C₁-C₄)Alkylamino, *N,N*-Di(C₁-C₄-alkyl)-amino, Sulfonyl, C₁-C₄-Alkylsulfonyl, Sulfinyl, C₁-C₄-Alkylsulfinyl ausgewählt sind und sich Heteroaryl auf aromatische und teilweise aromatische Gruppen eines monocyclischen oder bicyclischen Rings mit 4 bis 12 Kohlenstoffatomen bezieht und mindestens eines von ihnen ein Heteroatom ist, das aus der Gruppe bestehend aus O, S und N ausgewählt ist und wobei das Heteroaryl gegebenenfalls mit einem oder zwei Substituenten substituiert sein kann, welche aus Halogen, C₁-C₄-Alkyl, Cyano, Nitro, Hydroxy, C₁-C₄-Alkoxy, Thiol, C₁-C₄-Alkylthio, Amino, *N*-(C₁-C₄)Alkylamino, *N,N*-Di(C₁-C₄-alkyl)-amino, Sulfonyl, C₁-C₄-Alkylsulfonyl, Sulfinyl, C₁-C₄-Alkylsulfinyl ausgewählt sind;
m für eine ganze Zahl von 1 bis 3 steht;
Q für Sauerstoff, Schwefel oder NH steht;
und pharmazeutisch verträgliche Salze und Solvate davon.

2. Verbindung gemäß Anspruch 1, wobei X für O oder S steht.

3. Verbindung gemäß Anspruch 1, wobei Y und Z unabhängig voneinander für einen oder mehrere identische oder verschiedene Substituenten, die mit einem beliebigen verfügbaren Kohlenstoffatom verbunden sind, stehen, die aus der Gruppe bestehend aus Wasserstoff, Fluor, Chlor, Brom, C₁-C₄-Alkyl, Halogen-C₁-C₄-alkyl, Hydroxy, C₁-C₄-Alkoxy, Trifluormethoxy, C₁-C₄-Alkanoyl, Amino, Amino-C₁-C₄-alkyl, *N*-(C₁-C₄-Alkyl)amino, *N,N*-Di(C₁-C₄-alkyl)amino, Thiol, C₁-C₄-Alkylthio, Cyano und Nitro ausgewählt sind.

4. Verbindung gemäß Anspruch 1, wobei R¹ für Wasserstoff, Halogen, C₁-C₇-Alkyl, das gegebenenfalls substituiert ist mit einem, zwei, drei oder mehr Substituenten, die aus der Gruppe bestehend aus Halogenatom, Hydroxy, C₁-C₄-Alkoxy, Thiol, C₁-C₄-Alkylthio, Amino, *N*-(C₁-C₄)alkylamino und *N,N*-Di(C₁-C₄-alkyl)-amino ausgewählt sind; einen monocyclischen oder bicyclischen Arylrest, der 6 bis 10 Kohlenstoffatome und alternierende Doppelbindungen aufweist, und der Rest gegebenenfalls substituiert sein kann mit einem oder zwei Substituenten, die aus der Gruppe bestehend aus Fluor, Chlor, C₁-C₄-Alkyl, Cyano, Nitro, Hydroxy, C₁-C₄-Alkoxy, Thiol, C₁-C₄-Alkylthio, Amino, N-(C₁-C₄)Alkylamino und *N,N*-Di(C₁-C₄-alkyl)-amino ausgewählt sind und mit einem beliebigen verfügbaren Kohlenstoffatom über eine direkte Bindung oder mit einem C₁-C₄-Alkylenrest an den Rest des Moleküls gebunden sein können; monocyclisches oder bicyclisches Heteroaryl, das für aromatische und partiell aromatische Reste eines monocyclischen oder bicyclischen Rings mit 4 bis 12 Kohlenstoffatomen steht und mindestens eines von ihnen ein Heteroatom ist, das aus der Gruppe bestehend aus O, S und N ausgewählt ist, wobei verfügbarer Kohlenstoff oder Stickstoff die Bindungsstelle des Rests an den Rest des Moleküls entweder über direkte Bindung oder über einen C₁-C₄-Alkylenrest darstellt und wobei das Heteroaryl gegebenenfalls mit Fluor, Chlor, C₁-C₄-Alkyl, Cyano, Nitro, Hydroxy, C₁-C₄-Alkoxy, Thio!, C₁-C₄-Alkythio, Amino, *N*-(C₁-C₄)Alkylamino und *N,N*-Di(C₁-C₄-Alkyl)amino substituiert sein kann; einen fünfgliedrigen oder sechsgliedrigen völlig gesättigten oder teilweise ungesättigten heterocyclischen Rest, der mindestens ein Heteroatom enthält, das aus der Gruppe bestehend aus O, S und N ausgewählt ist, wobei verfügbarer Kohlenstoff oder Stickstoff die Bindungsstelle der Gruppe an den Rest des Moleküls entweder über direkte Bindung oder über einen C₁-C₄-Alkylenrest darstellt und wobei der Heterocyclus gegebenenfalls mit Fluor, Chlor, C₁-C₄-Alkyl, Cyano, Nitro, Hydroxy, C₁-C₄-Alkoxy, Thiol, C₁-C₄-Alkylthio, Amino, *N*-(C₁-C₄)Alkylamino und *N,N*-Di(C₁-C₄-alkyl)-amino substituiert sein kann; Hydroxy; C₁-C₇-Alkoxy; Thiol; C₁-C₇-Alkylthio; Amino; *N*-(C₁-C₇-Alkyl)amino; *N,N*-Di(C₁-C₇-alkyl)amino; Amino-C₁-C₇-alkoxy; C₁-C₇-Alkanoyl; C₇-C₁₀-Aroyl; C₁-C₇-Alkanoyloxy; ein C₁-C₇-Alkyloxycarbonyl, welches gegebenenfalls mit einem, zwei, drei oder mehr Substituenten substituiert ist, die ausgewählt sind aus der Gruppe bestehend aus Halogen, Hydroxy, C₁-C₄-Alkoxy, Thiol, C₁-C₄-Alkylthio, Amino, *N*-(C₁-C₄-Alkyl)amino, *N,N*-Di(C₁-C₄-alkyl)amino, Sulfonyl, C₁-C₄-Alkylsulfonyl, Sulfinyl und C₁-C₄-Alkylsulfinyl; C₇-C₁₀-Aryloxycarbonyl, welches gegebenenfalls mit einem oder zwei Substituenten substituiert ist, die ausgewählt sind aus der Gruppe bestehend aus Halogen, C₁-C₄-Alkyl, Cyano, Nitro, Hydroxy, C₁-C₄-Alkoxy, Thiol, C₁-C₄-Alkylthio, Amino, *N*-(C₁-C₄)Alkylamino, *N,N*-Di(C₁-C₄-Alkyl)amino, Sulfonyl, C₁-C₄-Alkylsulfonyl, Sulfinyl, C₁-C₄-Alkylsulfinyl, Carbamoyl; *N*-(C₁-C₇-Alkyl)carbamoyl; *N,N*-Di(C₁-C₇-alkyl)carbamoyl; Cyano, Cyano-C₁-C₇-alkyl; Nitro steht;
oder für einen Substituenten, der durch Formel **II** dargestellt ist: 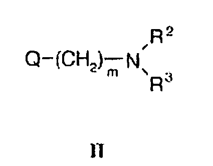 wobei
R² und R³ gleichzeitig oder unabhängig voneinander für Wasserstoff, C₁-C₄-Alkyl, Aryl, das die oben beschriebene Bedeutung hat, stehen; oder zusammen mit N für einen Heterocyclus oder Heteroaryl stehen, die aus der Gruppe bestehend aus Morpholin-4-yl, Piperidin-1-yl, Pyrrolidin-1-yl, Imidazol-1-yl und Piperazin-1-yl ausgewählt sind;
m für eine ganze Zahl von 1-3 steht;
Q für Sauerstoff steht.

5. Verbindung gemäß einem der Ansprüche 1 oder 3, wobei Y Wasserstoff oder Chlor darstellt und Z Wasserstoff darstellt.

6. Verbindung gemäß einem der Ansprüche 1 oder 4, wobei R¹ für CH₃, CH₂Br, CH₂OH oder einen Substituenten der Formel **II** steht: 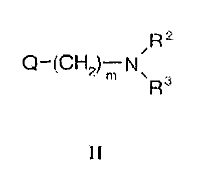 wobei R², R³, Q und m die oben definierte Bedeutung haben.

7. Verbindung gemäß Anspruch 6, wobei das Symbol m für 2 oder 3 steht.

8. Verbindung gemäß Anspruch 1, ausgewählt aus der Gruppe bestehend aus:
3-Methyl-2-oxa-8-thia-1-aza-dibenzo[*e,h*]azulen;
11-Chlor-3-methyl-2-oxa-8-thia-1-aza-dibenzo[*e,h*]azulen;
3-Methyl-2,8-dioxa-1-aza-dibenzo[*e,h*]azulen;
3-Brommethyl-2-oxa-8-thia-1-aza-dibenzo[*e,h*]azulen;
3-Brommethyl-11-chlor-2-oxa-8-thia-1-aza-dibenzo[*e,h*]azulen;
3-Brommethyl-2,8-dioxa-1-aza-dibenzo[*e,h*]azulen;
Dimethyl-[2-(2-oxa-8-thia-1-aza-dibenzo[*e,h*]azulen-3-ylmethoxy)-ethyl]-amin;
Dimethyl-[3-(2-oxa-8-thia-1-aza-dibenzo[*e,h*]azulen-3-ylmethoxy)-propyl]-amin;
Dimethyl-[2-(11-chlor-2-oxa-8-thia-1-aza-dibenzo[*e,h*]azulen-3-ylmethoxy)ethyl]amin;
Dimethyl-[3-(11-chlor-2-oxa-8-thia-1-aza-dibenzo[*e,h*]azulen-3-ylmethoxy)-propyl]-amin;
Dimethyl-[2-(2,8-dioxa-1-aza-dibenzo[*e,h*]azulen-3-ylmethoxy)-ethyl]-amin; und
Dimethyl-[3-(2,8-dioxa-1-aza-dibenzo[*e,h*]azulen-3-ylmethoxy)-propyl]-amin.

9. Verfahren zur Herstellung der Verbindung der Formel **I**: 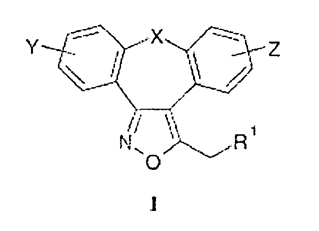 wobei
X für CH₂ oder ein Heteroatom steht, das aus der Gruppe bestehend aus O, S, S(=O), S(=O)₂ und NR^{a} ausgewählt ist, wobei R^{a} Wasserstoff ist oder ein Substituent, der aus der Gruppe bestehend aus C₁-C₃-Alkyl, C₁-C₃-Alkanoyl, C₁-C₇-Alkoxycarbonyl, C₇-C₁₀-Arylalkyloxycarbonyl, C₇-C₁₀-Aroyl, C₇-C₁₀-Arylalkyl, C₃-C₇-Alkylsilyl und C₅-C₁₀-Alkylsilylalkyloxyalkyl ausgewählt ist;
Y und Z unabhängig voneinander für einen oder mehrere identische oder verschiedene Substituenten stehen, die an ein beliebiges verfügbares Kohlenstoffatom gebunden sind, und die aus der Gruppe bestehend aus Wasserstoff, Halogen, C₁-C₄-Alkyl, C₂-C₄-Alkenyl, C₂-C₄-Alkinyl, Halogen-C₁-C₄-alkyl, Hydroxy, C₁-C₄-Alkoxy, Trifluormethoxy, C₁-C₄-Alkanoyl, Amino, Amino-C₁-C₄-alkyl, C₁-C₄-Alkylamino, *N*-(C₁-C₄-Alkyl)amino, *N,N*-Di(C₁-C₄-alkyl)amino, Thiol, C₁-C₄-Alkylthio, Sulfonyl, C₁-C₄-Alkylsulfonyl, Sulfinyl, C₁-C₄-Alkylsulfinyl, Carboxy, C₁-C₄-Alkoxycarbonyl, Cyano und Nitro ausgewählt sind;
R¹ für Wasserstoff, Halogen, gegebenenfalls substituiertes C₁-C₇-Alkyl, das gegebenenfalls mit einem, zwei, drei oder mehr Substituenten, die aus der Gruppe bestehend aus Halogenatom, Hydroxy, C₁-C₄-Alkoxy, Thiol, C₁-C₄-Alkylthio, Amino, *N*-(C₁-C₄)alkylamino, *N*,*N*-Di(C₁-C₄-alkyl)-amino, Sulfonyl, C₁-C₄-Alkylsulfonyl, Sulfinyl und C₁-C₄-Alkylsulfinyl ausgewählt sind, substituiert ist; C₂-C₇-Alkenyl, welches gegebenenfalls mit einem, zwei, drei oder mehr Halogenatomen substituiert ist; C₂-C₇-Alkinyl; einen monocyclischen oder bicyclischen Arylrest, der 6 bis 10 Kohlenstoffatome und alternierende Doppelbindungen hat und die gegebenenfalls mit einem oder zwei Substituenten, die aus der Gruppe bestehend aus Fluor, Chlor, C₁-C₄-Alkyl, Cyano, Nitro, Hydroxy, C₁-C₄-Alkoxy, Thiol, C₁-C₄-Alkylthio, Amino, *N*-(C₁-C₄)Alkylamino, *N,N*-Di(C₁-C₄-alkyl)-amino, Sulfonyl, C₁-C₄-Alkylsulfonyl, Sulfinyl, C₁-C₄-Alkylsulfinyl ausgewählt sind, substituiert ist und mit einem beliebigen verfügbaren Kohlenstoffatom über direkte Bindung oder mit einem C₁-C₄-Alkylenrest an den Rest des Moleküls gebunden sein kann; ein monocyclisches oder bicyclisches Heteroaryl, das für aromatische und partiell aromatische Reste eines monocyclischen oder bicyclischen Rings mit 4 bis 12 Kohlenstoffatomen steht und mindestens eines von ihnen ein Heteroatom ist, das aus der Gruppe bestehend aus O, S und N ausgewählt ist, wobei verfügbarer Kohlenstoff oder Stickstoff die Bindungsstelle des Restes zum Rest des Moleküls entweder über eine direkte Bindung oder einen C₁-C₄-Alkylenrest darstellt und wobei das Heteroaryl gegebenenfalls mit Fluor, Chlor, C₁-C₄-Alkyl, Cyano, Nitro, Hydroxy, C₁-C₄-Alkoxy, Thiol, C₁-C₄-Alkylthio, Amino, *N*-(C₁-C₄)Alkylamino, *N,N*-Di(C₁-C₄-alkyl)-amino, Sulfonyl, C₁-C₄-Alkylsulfonyl, Sulfinyl, C₁-C₄-Alkylsulfinyl substituiert sein kann; einen fünfgliedrigen oder sechsgliedrigen völlig gesättigten oder teilweise ungesättigten heterocyclischen Rest, der mindestens ein Heteroatom, das aus der Gruppe bestehend aus O, S und N ausgewählt ist, enthält, wobei verfügbarer Kohlenstoff oder Stickstoff die Bindungsstelle des Rests an den Rest des Moleküls entweder über eine direkte Bindung oder über einen C₁-C₄-Alkylenrest darstellt und wobei der Heterocyclus gegebenenfalls mit Fluor, Chlor, C₁-C₄-Alkyl, Cyano, Nitro, Hydroxy, C₁-C₄-Alkoxy, Thiol, C₁-C₄-Alkylthio, Amino, *N*-(C₁-C₄)Alkylamino, *N,N*-Di(C₁-C₄-alkyl)-amino, Sulfonyl, C₁-C₄-Alkylsulfonyl, Sulfinyl, C₁-C₄-Alkylsulfinyl substituiert sein kann; Hydroxy; Hydroxy-C₂-C₇-alkenyl; Hydroxy-C₂-C₇-alkinyl; C₁-C₇-Alkoxy; Thiol; Thio-C₂-C₇-alkenyl; Thio-C₂-C₇-alkinyl; C₁-C₇-Alkylthio; Amino; *N*-(C₁-C₇-alkyl)amino; *N,N*-Di(C₁-C₇-alkyl)amino; C₁-C₇-Alkylamino; Amino-C₂-C₇-alkenyl; Amino-C₂-C₇-alkinyl; Amino-C₁-C₇-alkoxy; C₁-C₇-Alkanoyl; C₇-C₁₀-Aroyl; Oxo-C₁-C₇-alkyl; C₁-C₇-Alkanoyloxy; Carboxy; C₁-C₇-Alkyloxycarbonyl, welches gegebenenfalls mit einem, zwei, drei oder mehr Substituenten substituiert ist, die ausgewählt sind aus der Gruppe bestehend aus Halogen, Hydroxy, C₁-C₄-Alkoxy, Thiol, C₁-C₄-Alkylthio, Amino, *N*-(C₁-C₄-Alkyl)amino, *N,N*-Di(C₁-C₄-alkyl)amino, Sulfonyl, C₁-C₄-Alkylsulfonyl, Sulfinyl und C₁-C₄-Alkylsulfinyl; C₇-C₁₀-Aryloxycarbonyl, welches gegebenenfalls substituiert ist mit einem oder zwei Substituenten, die ausgewählt sind aus der Gruppe bestehend aus Halogen, C₁-C₄-Alkyl, Cyano, Nitro, Hydroxy, C₁-C₄-Alkoxy, Thiol, C₁-C₄-Alkylthio, Amino, *N*-(C₁-C₄)Alkylamino, *N*,*N*-Di(C₁-C₄-alkyl)amino, Sulfonyl, C₁-C₄-Alkylsulfonyl, Sulfinyl, C₁-C₄-Alkylsulfinyl; *N*-(C₁-C₇-Alkyl)carbamoyl; *N*,*N*-Di(C₁-C₇-alkyl)carbamoyl; Cyano; Cyano-C₁-C₇-alkyl; Sulfonyl; C₁-C₇-Alkylsulfonyl; Sulfinyl; C₁-C₇-Alkylsulfinyl; Nitro steht;
oder für einen Substituenten, der durch Formel **II** dargestellt ist: 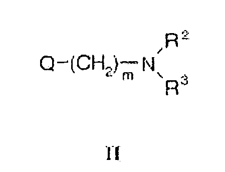
wobei
R² und R³ gleichzeitig oder unabhängig voneinander für Wasserstoff, C₁-C₄-Alkyl, Aryl, das die oben definierte Bedeutung hat, stehen, oder zusammen mit N für einen gegebenenfalls substituierten Heterocyclus oder Heteroaryl stehen, wobei sich der Heterocyclus auf einen fünfgliedrigen oder sechsgliedrigen völlig gesättigten oder teilweise ungesättigten Heterocyclusrest bezieht, der mindestens ein Heteroatom enthält, das aus der Gruppe bestehend aus O, S und N ausgewählt ist und wobei der Heterocyclus gegebenenfalls mit einem oder zwei Substituenten substituiert sein kann, welche aus Halogen, C₁-C₄-Alkyl, Cyano, Nitro, Hydroxy, C₁-C₄-Alkoxy, Thiol, C₁-C₄-Alkylthio, Amino, *N*-(C₁-C₄)Alkylamino, *N,N*-Di(C₁-C₄-alkyl)-amino, Sulfonyl, C₁-C₄-Alkylsulfonyl, Sulfinyl, C₁-C₄-Alkylsulfinyl ausgewählt sind; und sich Heteroaryl auf aromatische oder teilweise aromatische Reste eines monocyclischen oder bicyclischen Rings mit 4 bis 12 Kohlenstoffatomen bezieht und mindestens eines von ihnen ein Heteroatom ist, das aus der Gruppe bestehend aus O, S und N ausgewählt ist und wobei das Heteroaryl gegebenenfalls mit einem oder zwei Substituenten substituiert sein kann, welche aus Halogen, C₁-C₄-Alkyl, Cyano, Nitro, Hydroxy, C₁-C₄-Alkoxy, Thiol, C₁-C₄-Alkylthio, Amino, *N*-(C₁-C₄)Alkylamino, *N,N*-Di(C₁-C₄-alkyl)-amino, Sulfonyl, C₁-C₄-Alkylsulfonyl, Sulfinyl, C₁-C₄-Alkylsulfinyl ausgewählt sind;
m für eine ganze Zahl von 1 bis 3 steht; und
Q für Sauerstoff, Schwefel oder NH steht;
und ihrer pharmakologisch verträglichen Salze und Solvate,
welches umfasst:
a) Kondensation einer Verbindung **Ia**: 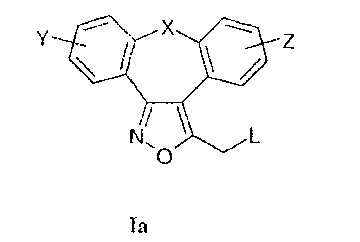 wobei die Symbole X, Y und Z die oben definierte Bedeutung haben, L für eine Abgangsgruppe steht,
mit einem wahlweise ausgewählten Alkohol, Thioalkohol oder Amin oder mit einer Verbindung der Formel **IIa**: 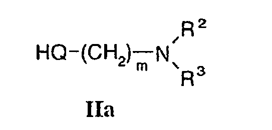 wobei alle Reste und Symbole die früher angegebenen Bedeutungen haben; oder
b) Kondensation einer Verbindung der Formel **Ib:** 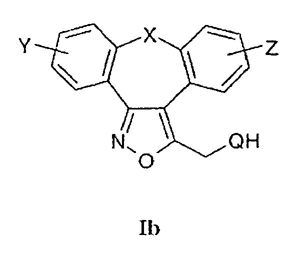
wobei alle Symbole die früher angegebenen Bedeutungen haben, mit einer Verbindung der Formel IIb: 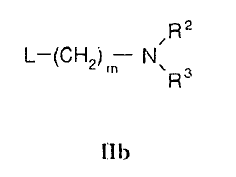 wobei die Reste R² und R³ und das Symbol m die früher angegebenen Bedeutungen haben und das Symbol L für eine geeignete Abgangsgruppe steht.

10. Arzneimittel, das mindestens eine Verbindung gemäß Anspruch 1 und ein pharmazeutisch verträgliches Salz oder Solvat davon in Verbindung mit einem pharmazeutisch verträglichen Exzipienten, Verdünnungsmittel und/oder Träger umfasst.

11. Verwendung einer Verbindung gemäß Anspruch 1 zur Herstellung einer pharmazeutischen Formulierung zur Behandlung und Prävention von Krankheiten, Schäden und Störungen des zentralen Nervensystems, die durch Störungen des neurochemischen Gleichgewichts biogener Amine oder anderer Neurotransmitter verursacht werden.

12. Verwendung gemäß Anspruch 11, wobei die ausgewählten biogenen Amine Serotonin, Norepinephrin und Dopamin sind.

13. Verwendung gemäß Anspruch 11, wobei der Neurotransmitter Glutamat ist.

14. Verwendung gemäß den Ansprüchen 11, 12 oder 13, wobei die Verbindungen der allgemeinen Formel I auf das neurochemische Gleichgewicht durch Regulierung der Synthese, durch Speichern, Abgabe, Metabolisierung und/oder Resorption von biogenen Aminen oder Neurotransmittern und Bindung an ihre Rezeptoren einwirken.

15. Verwendung gemäß Anspruch 14, wobei die Verbindungen der allgemeinen Formel **I** Bindungsaffinität zu einem Rezeptor eines oder mehrerer biogener Amine zeigen.

16. Verwendung gemäß Anspruch 15, wobei die Verbindungen der allgemeinen Formel **I** signifikante Bindungsaffinität zu Serotonin-5-HT_{2A}- und 5-HT_{2C}-Rezeptoren zeigen.

17. Verwendung gemäß Anspruch 16, wobei die Verbindungen der allgemeinen Formel **I** Bindungsaffinität zu ausgewählten Serotoninrezeptoren in einer Konzentration von IC₅₀<1µM zeigen.

18. Verwendung gemäß Anspruch 11, wobei die Verbindungen der allgemeinen Formel **I** als σ1 Rezeptorliganden in einer Konzentration von IC₅₀<1µM wirken, indem sie ein zentrales Neurotransmittersystem modulieren.

19. Verwendung gemäß den Ansprüchen 11, 16 oder 18, wobei die Verbindungen der allgemeinen Formel I duale Bindungsaffinität zu einem σ1 Rezeptor und zu mindestens einem Serotoninrezeptor, der aus 5-HT_{2A}- und 5-HT_{2C} ausgewählt ist, zeigen.

20. Verwendung gemäß Anspruch 11, wobei die Krankheiten und Störungen des zentralen Nervensystems aus der Gruppe bestehend aus Angst, Depression und mittelschwerer Depression, bipolaren Störungen, Schlafstörungen, sexuellen Störungen, Psychose, Borderline-Psychose, Schizophrenie, Migräne, Persönlichkeitsstörungen und Zwangsneurosen (OCD), sozialer Phobie oder Panikattacken, organischen psychischen Störungen bei Kindern, Aggression, Gedächtnisstörungen und Persönlichkeitsstörungen bei älteren Personen, Sucht, Fettsucht, Bulimie und ähnlichen Störungen, Schnarchen, prämenstruellen Beschwerden ausgewählt sind.

21. Verwendung gemäß Anspruch 11, wobei die Schäden des zentralen Nervensystems durch Trauma, Gehirnschlag, neurodegenerative Krankheiten, Herz-Kreislauf-Störungen wie hoher Blutdruck, Thrombose, Infarkt wie auch Magen-Darm-Störungen verursacht werden.

22. Verwendung gemäß Anspruch 11, wobei die Verbindungen der allgemeinen Formel **I,** pharmazeutisch verträgliche Salze und Solvate davon ausgewählt sind aus der Gruppe bestehend aus:
3-Methyl-2-oxa-8-thia-1-aza-dibenzo[*e,h*]azulen;
11-Chlor-3-methyl-2-oxa-8-thia-1-aza-dibenzo[*e,h*]azulen;
3-Methyl-2,8-dioxa-1-aza-dibenzo[*e,h*]azulen;
3-Brommethyl-2-oxa-8-thia-1-aza-dibenzo[*e,h*]azulen;
3-Brommethyl-11-chlor-2-oxa-8-thia-1-aza-dibenzo[*e,h*]azulen;
3-Brommethyl-2,8-dioxa-1-aza-dibenzo[*e,h*]azulen;
Dimethyl-[2-(2-oxa-8-thia-1-aza-dibenzo[*e,h*]azulen-3-ylmethoxy)-ethyl]-amin;
Dimethyl-[3-(2-oxa-8-thia-1-aza-dibenzo[*e,h*]azulen-3-ylmethoxy)-propyl]-amin;
Dimethyl-[2-(11-chlor-2-oxa-8-thia-1-aza-dibenzo[*e,h*]azulen-3-ylmethoxy)ethyl]amin;
Dimethyl-[3-(11-chlor-2-oxa-8-thia-1-aza-dibenzo[*e,h*]azulen-3-ylmethoxy)-propyl]-amin;
Dimethyl-[2-(2,8-dioxa-1-aza-dibenzo[*e,h*]azulen-3-ylmethoxy)-ethyl]-amin; und
Dimethyl-[3-(2,8-dioxa-1-aza-dibenzo[*e,h*]azulen-3-ylmethoxy)-propyl]-amin.

## Revendications

1. Composé de formule I : 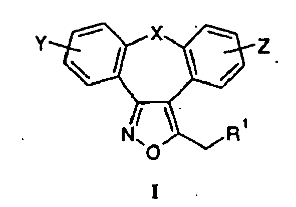 dans laquelle
X représente un groupe CH₂ ou un hétéroatome choisi dans le groupe consistant en O, S, S(=O), S(=O)₂ et NR^{a}, dans lequel R^{a} représente un atome d'hydrogène ou un substituant choisi dans le groupe consistant en des substituants alkyle en C₁ à C₃, alcanoyle en C₁ à C₃, (alkoxy en C₁ à C₇)carbonyle, (aryle en C₇ à C₁₀)-alkyloxycarbonyle, aroyle en C₇ à C₁₀, (aryle en C₇ à C₁₀)-alkyle, alkylsilyle en C₃ à C₇ et (alkyle en C₅ à C₁₀)-silylalkyloxyalkyle ;
Y et Z, indépendamment l'un de l'autre, représentent un ou plusieurs substituants identiques ou différents liés à n'importe quel atome de carbone disponible, choisis dans le groupe consistant en un atome d'hydrogène, un atome d'halogène, des groupes alkyle en C₁ à C₄, alcényle en C₂ à C₄, alcynyle en C₂ à C₄, halogénalkyle en C₁ à C₄, hydroxy, alkoxy en C₁ à C₄, trifluorométhoxy, alcanoyle en C₁ à C₄, amino, aminoalkyle en C₁ à C₄, alkylamino en C₁ à C₄, *N-*(alkyle en C₁ à C₄)amino, *N,N*-di(alkyle en C₁ à C₄)-amino, thiol, alkylthio en C₁ à C₄, sulfonyle, alkylsulfonyle en C₁ à C₄, sulfinyle, alkylsulfinyle en C₁ à C₄, carboxy, (alkoxy en C₁ à C₄)carbonyle, cyano et nitro ;
R¹ représente un atome d'hydrogène, un atome d'halogène, un groupe alkyle en C₁ à C₇ facultativement substitué avec un, deux, trois ou plus de trois substituants choisis dans le groupe consistant en un atome d'halogène, des substituants hydroxy, alkoxy en C₁ à C₄, thiol, alkylthio en C₁ à C₄, amino, N-(alkyle en C₁ à C₄)-amino, N,N-di(alkyle en C₁ à C₄)amino, sulfonyle, alkylsulfonyle en C₁ à C₄, sulfinyle et alkylsulfonyle en C₁ à C₄; alcényle en C₂ à C₇ facultativement substitué avec un, deux, trois ou plus de trois atomes d'halogène; alcynyle en C₂ à C₇; un groupe aryle monocyclique ou bicyclique ayant 6 à 10 atomes de carbone et des doubles liaisons alternées, ledit groupe pouvant être facultativement substitué avec un ou deux substituants choisis dans le groupe consistant en des substituants fluoro, chloro, alkyle en C₁ à C₄, cyano, nitro, hydroxy, alkoxy en C₁ à C₄, thiol, alkylthio en C₁ à C₄, amino, *N*-(alkyle en C₁ à C₄)-amino, *N,N*-di(alkyle en C₁ à C₄)amino, sulfonyle, alkylsulfonyle en C₁ à C₄, sulfinyle, alkylsulfinyle en C₁ à C₄ et pouvant être lié au reste de la molécule par n'importe quel atome de carbone disponible par une liaison directe ou par l'intermédiaire d'un groupe alkylène en C₁ à C₄; un groupe hétéroaryle monocyclique ou bicyclique correspondant à des groupes aromatiques et partiellement aromatiques d'un noyau monocyclique ou bicyclique ayant 4 à 12 atomes de carbone dont au moins un atome est un hétéroatome choisi dans le groupe consistant en O, S et N, où l'atome de carbone ou d'azote disponible représente le site de liaison du groupe au reste de la molécule par une liaison directe ou par l'intermédiaire d'un groupe alkylène en C₁ à C₄, et où ledit groupe hétéroaryle peut être facultativement substitué avec des substituants fluoro, chloro, alkyle en C₁ à C₄, cyano, nitro, hydroxy, alkoxy en C₁ à C₄, thiol, alkylthio en C₁ à C₄, amino, *N*-(alkyle en C₁ à C₄)amino, *N*,*N-*di(alkyle en C₁ à C₄)amino, sulfonyle, alkylsulfonyle en C₁ à C₄, sulfinyle, alkylsulfinyle en C₁ à C₄ ; un groupe hétérocyclique pentagonal ou hexagonal, totalement saturé ou partiellement insaturé, contenant au moins un hétéroatome choisi dans le groupe consistant en O, S et N, dans lequel l'atome de carbone ou d'azote disponible représente le site de liaison du groupe au reste de la molécule par une liaison directe ou par l'intermédiaire d'un groupe alkylène en C₁ à C₄, et où ledit hétérocycle peut être facultativement substitué avec des substituants fluoro, chloro, alkyle en C₁ à C₄, cyano, nitro, hydroxy, alkoxy en C₁ à C₄, thiol, alkylthio en C₁ à C₄, amino, *N*-(alkyle en C₁ à C₄)amino, *N,N*-di(alkyle en C₁ à C₄)amino, sulfonyle, alkylsulfonyle en C₁ à C₄, sulfinyle, alkylsulfinyle en C₁ à C₄ ; un groupe hydroxy ; hydroxyalcényle en C₂ à C₇ ; hydroxyalcynyle en C₂ à C₇ ; alkoxy en C₁ à C₇ ; thiol ; thioalcényle en C₂ à C₇ ; thioalcynyle en C₂ à C₇ ; alkylthio en C₁ à C₇ ; amino ; *N*-(alkyle en C₁ à C₇)amino ; *N,N-*di(alkyle en C₁ à C₇)amino ; alkylamino en C₁ à C₇ ; aminoalcényle en C₂ à C₇ ; aminoalcynyle en C₂ à C₇ ; aminoalkoxy en C₁ à C₇ ; alcanoyle en C₁ à C₇ ; aroyle en C₇ à C₁₀ ; oxoalkyle en C₁ à C₇ ; alcanoyloxy en C₁ à C₇ ; carboxy ; (alkyle en C₁ à C₇)oxycarbonyle, qui est facultativement substitué avec un, deux, trois ou plus de trois substituants choisis dans le groupe consistant en des substituants halogéno, hydroxy, alkoxy en C₁ à C₄, thiol, alkylthio en C₁ à C₄, amino, *N*-(alkyle en C₁ à C₄)amino, *N,N*-di(alkyle en C₁ à C₄)-amino, sulfonyle, alkylsulfonyle en C₁ à C₄, sulfinyle et alkylsulfinyle en C₁ à C₄ ; (aryle en C₇ à C₁₀)-oxycarbonyle, qui est facultativement substitué avec un ou deux substituants choisis dans le groupe consistant en des substituants halogéno, alkyle en C₁ à C₄, cyano, nitro, hydroxy, alkoxy en C₁ à C₄, thiol, alkylthio en C₁ à C₄, amino, N-(alkyle en C₁ à C₄)-amino, *N,N*-di(alkyle en C₁ à C₄)amino, sulfonyle, alkylsulfonyle en C₁ à C₄, sulfinyle, alkylsulfinyle en C₁ à C₄ ; *N*-(alkyle en C₁ à C₇)carbamoyle ; *N,N-*di(alkyle en C₁ à C₇)carbamoyle ; cyano ; cyano(alkyle en C₁ à C₇) ; sulfonyle ; alkylsulfonyle en C₁ à C₇ ; sulfinyle ; alkylsulfinyle en C₁ à C₇ ; nitro ;
ou un substituant représenté par la formule II : 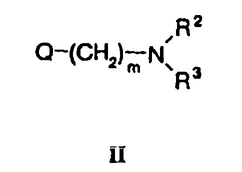
dans laquelle
R² et R³, simultanément ou indépendamment l'un de l'autre, représentent un atome d'hydrogène, un groupe alkyle en C₁ à C₄, ou un groupe aryle répondant à la définition précitée ou bien, conjointement avec N, représentent un groupe hétérocyclique ou hétéroaryle facultativement substitué, le terme "hétérocycle" désignant un groupe hétérocyclique pentagonal ou hexagonal totalement saturé ou partiellement insaturé contenant au moins un hétéroatome choisi dans le groupe consistant en O, S et N, ledit hétérocycle pouvant être facultativement substitué avec un ou deux substituants qui sont choisis entre des substituants halogéno, alkyle en C₁ à C₄, cyano, nitro, hydroxy, alkoxy en C₁ à C₄, thiol, alkylthio en C₁ à C₄, amino, *N*-(alkyle en C₁ à C₄)-amino, *N,N*-di(alkyle en C₁ à C₄)amino, sulfonyle, alkylsulfonyle en C₁ à C₄, sulfinyle, alkylsulfinyle en C₁ à C₄, et le terme "hétéroaryle" désigne des groupes aromatiques et partiellement aromatiques d'un noyau monocyclique ou bicyclique ayant 4 à 12 atomes de carbone dont au moins un des atomes est un hétéroatome choisi dans le groupe consistant en O, S et N, ledit groupe hétéroaryle pouvant être facultativement substitué avec un ou deux substituants qui sont choisis entre des substituants halogéno, alkyle en C₁ à C₄, cyano, nitro, hydroxy, alkoxy en C₁ à C₄, thiol, alkylthio en C₁ à C₄, amino, *N*-(alkyle en C₁ à C₄)amino, *N,N-*di(alkyle en C₁ à C₄)amino, sulfonyle, alkylsulfonyle en C₁ à C₄, sulfinyle et alkylsulfinyle en C₁ à C₄ ;
m représente un nombre entier de 1 à 3 ;
Q représente un atome d'oxygène, un atome de soufre ou un groupe NH ;
et ses sels et produits de solvatation pharmaceutiquement acceptables.

2. Composé suivant la revendication 1, dans lequel X représente un atome de O ou S.

3. Composé suivant la revendication 1, dans lequel Y et Z, indépendamment l'un de l'autre, représentent un ou plusieurs substituants identiques ou différents liés à n'importe quel atome de carbone disponible, choisis dans le groupe consistant en des atomes d'hydrogène, de fluor, de chlore et de brome, des groupes alkyle en C₁ à C₄, halogénalkyle en C₁ à C₄, hydroxy, alkoxy en C₁ à C₄, trifluorométhoxy, alcanoyle en C₁ à C₄, amino, aminoalkyle en C₁ à C₄, *N*-(alkyle en C₁ à C₄)amino, *N,N*-di(alkyle en C₁ à C₄)amino, thiol, alkylthio en C₁ à C₄, cyano et nitro.

4. Composé suivant la revendication 1, dans lequel R¹ représente un atome d'hydrogène, un atome d'halogène, un groupe alkyle en C₁ à C₇ facultativement substitué avec un, deux ou trois ou plus de trois substituants choisis dans le groupe consistant en un atome d'halogène, des substituants hydroxy, alkoxy en C₁ à C₄, thiol, alkylthio en C₁ à C₄, amino, *N*-(alkyle en C₁ à C₄)amino et *N,N*-di(alkyle en C₁ à C₄)amino ; un groupe aryle monocyclique ou bicyclique comprenant 6 à 10 atomes de carbone et des doubles liaisons alternées, et ledit groupe pouvant être facultativement substitué avec un ou deux substituants choisis dans le groupe consistant en des substituants fluoro, chloro, alkyle en C₁ à C₄, cyano, nitro, hydroxy, alkoxy en C₁ à C₄, thiol, alkylthio en C₁ à C₄, amino, *N*-(alkyle en C₁ à C₄)amino et *N,N*-di(alkyle en C₁ à C₄)amino et pouvant être lié au reste de la molécule par n'importe quel atome de carbone disponible par une liaison directe ou par l'intermédiaire d'un groupe alkylène en C₁ à C₄ ; un groupe hétéroaryle monocyclique ou bicyclique représentant des groupes aromatiques et partiellement aromatiques d'un noyau monocyclique ou bicyclique ayant 4 à 12 atomes de carbone dont au moins un est un hétéroatome choisi dans le groupe consistant en O, S et N, où l'atome de carbone ou d'azote disponible représente le site de liaison du groupe au reste de la molécule par une liaison directe ou par l'intermédiaire d'un groupe alkylène en C₁ à C₄, et où ledit groupe hétéroaryle peut être facultativement substitué avec des substituants fluoro, chloro, alkyle en C₁ à C₄, cyano, nitro, hydroxy, alkoxy en C₁ à C₄, thiol, alkylthio en C₁ à C₄, amino, *N*- (alkyle en C₁ à C₄)amino et *N,N*-di(alkyle en C₁ à C₄)amino ; un groupe hétérocyclique pentagonal ou hexagonal totalement saturé ou partiellement insaturé contenant au moins un hétéroatome choisi dans le groupe consistant en O, S et N, où l'atome de carbone ou d'azote disponible représente le site de liaison du groupe au reste de la molécule par une liaison directe ou par l'intermédiaire d'un groupe alkylène en C₁ à C₄, et où ledit hétérocycle peut être facultativement substitué avec des substituants fluoro, chloro, alkyle en C₁ à C₄, cyano, nitro, hydroxy, alkoxy en C₁ à C₄, thiol, alkylthio en C₁ à C₄, amino, *N*-(alkyle en C₁ à C₄)amino et *N,N*-di(alkyle en C₁ à C₄)amino ; un groupe hydroxy ; alkoxy en C₁ à C₇ ; thiol ; alkylthio en C₁ à C₇ ; amino ; *N*-(alkyle en C₁ à C₇)amino ; *N,N*-di(alkyle en C₁ à C₇)amino ; aminoalkoxy en C₁ à C₇ ; alcanoyle en C₁ à C₇ ; aroyle en C₇ à C₁₀ ; alcanoyloxy en C₁ à C₇ ; un groupe (alkyle en C₁ à C₇)oxycarbonyle, qui est facultativement substitué avec un, deux, trois ou plus de trois substituants choisis dans le groupe consistant en des substituants halogéno, hydroxy, alkoxy en C₁ à C₄, thiol, alkylthio en C₁ à C₄, amino, N-(alkyle en C₁ à C₄)amino, N,N-di(alkyle en C₁ à C₄)amino, sulfonyle, alkylsulfonyle en C₁ à C₄, sulfinyle et alkylsulfinyle en C₁ à C₄ ; un groupe (aryle en C₇ à C₁₀)oxycarbonyle, qui est facultativement substitué avec un ou deux substituants choisis dans le groupe consistant en des substituants halogéno, alkyle en C₁ à C₄, cyano, nitro, hydroxy, alkoxy en C₁ à C₄, thiol, alkylthio en C₁ à C₄, amino, N-(alkyle en C₁ à C₄)amino, N,N-di(alkyle en C₁ à C₄)amino, sulfonyle, alkylsulfonyle en C₁ à C₄, sulfinyle, alkylsulfinyle en C₁ à C₄, carbamoyle ; un groupe *N*-(alkyle en C₁ à ₇)carbamoyle ; *N,N*-di(alkyle en C₁ à C₇)carbamoyle ; cyano ; cyano(alkyle en C₁ à C₇) ; ou nitro ;
ou un substituant représenté par la formule II : 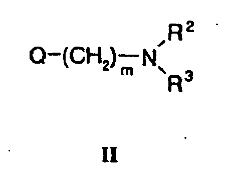 dans laquelle
R² et R³, simultanément ou indépendamment l'un de l'autre, représentent un atome d'hydrogène, un groupe alkyle en C₁ à C₄ ou un groupe aryle répondant à la définition précitée ; ou bien, conjointement avec N, représentent un hétérocycle ou un groupe hétéroaryle choisi dans le groupe consistant en les groupes morpholine-4-yle, pipéridine-1-yle, pyrrolidine-1-yle, imidazole-1-yle et pipérazine-1-yle ;
m représente un nombre entier de 1 à 3 ;
Q représente un atome d'oxygène.

5. Composé suivant la revendication 1 ou 3, dans lequel Y représente un atome d'hydrogène ou un atome de chlore et Z représente un atome d'hydrogène.

6. Composé suivant la revendication 1 ou 4, dans lequel R¹ représente un groupe CH₃, CH₂Br, CH₂OH ou un substituant de formule II : 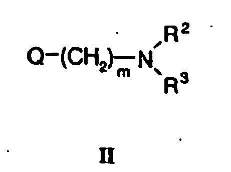 dans laquelle R², R³, Q et m répondent aux définitions précitées.

7. Composé suivant la revendication 6, dans lequel le symbole m est égal à 2 ou 3.

8. Composé suivant la revendication 1, choisi dans le groupe consistant en :
le 3-méthyl-2-oxa-8-thia-1-aza-dibenzo[*e,h*]azulène ;
le 11-chloro-3-méthyl-2-oxa-8-thia-1-aza-dibenzo[*e,h*]-azulène ;
le 3-méthyl-2,8-dioxa-1-aza-dibenzo[*e,h*]azulène ;
le 3-bromométhyl-2-oxa-8-thia-1-aza-dibenzo[*e,h*]azulène ;
le 3-bromométhyl-11-chloro-2-oxa-8-thia-1-aza-dibenzo-[*e,h*]azulène ;
le 3-bromométhyl-2,8-dioxa-1-aza-dibenzo[*e,h*]azulène ;
la diméthyl-[2-(2-oxa-8-thia-1-aza-dibenzo[*e,h*]azulène-3-ylméthoxy)-éthyl]-amine ;
la diméthyl-[3-(2-oxa-8-thia-1-aza-dibenzo[*e,h*]azulène-3-ylméthoxy)-propyl]-amine ;
la diméthyl-[2-(11-chloro-2-oxa-8-thia-1-aza-dibenzo-[*e,h*]azulène-3-ylméthoxy)-éthyl]-amine ;
la diméthyl-[3-(11-chloro-2-oxa-8-thia-1-aza-dibenzo-[*e,h*]azulène-3-ylméthoxy)-propyl]-amine ;
la diméthyl-[2-(2,8-dioxa-1-aza-dibenzo[*e,h*]azulène-3-ylméthoxy)-éthyl]-amine ; et
la diméthyl-[3-(2,8-dioxa-1-aza-dibenzo[*e,h*]azulène-3-ylméthoxy)-propyl]-amine.

9. Procédé de préparation du composé de formule I : 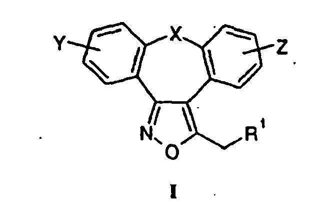 dans laquelle
X représente un groupe CH₂ ou un hétéroatome choisi dans le groupe consistant en O, S, S(=O), S(=O)₂ et NR^{a}, dans lequel R^{a} représente un atome d'hydrogène ou un substituant choisi dans le groupe consistant en des substituants alkyle en C₁ à C₃, alcanoyle en C₁ à C₃, (alkoxy en C₁ à C₇)carbonyle, (aryle en C₇ à C₁₀)-alkyloxycarbonyle, aroyle en C₇ à C₁₀, (aryle en C₇ à C₁₀)-alkyle, alkylsilyle en C₃ à C₇ et (alkyle en C₅ à C₁₀)-silylalkyloxyalkyle ;
Y et Z, indépendamment l'un de l'autre, représentent un ou plusieurs substituants identiques ou différents liés à n'importe quel atome de carbone disponible, choisis dans le groupe consistant en un atome hydrogène, un atome d'halogène, des groupes alkyle en C₁ à C₄, alcényle en C₂ à C₄, alcynyle en C₂ à C₄, halogénalkyle en C₁ à C₄, hydroxy, alkoxy en C₁ à C₄, trifluorométhoxy, alcanoyle en C₁ à C₄, amino, aminoalkyle en C₁ à C₄, alkylamino en C₁ à C₄, *N-*(alkyle en C₁ à C₄)amino, *N,N*-di(alkyle en C₁ à C₄)-amino, thiol, alkylthio en C₁ à C₄, sulfonyle, alkylsulfonyle en C₁ à C₄, sulfinyle, alkylsulfinyle en C₁ à C₄, carboxy, (alkoxy en C₁ à C₄)carbonyle, cyano et nitro ;
R¹ représente un atome d'hydrogène, un atome d'halogène, un groupe alkyle en C₁ à C₇ facultativement substitué avec un, deux, trois ou plus de trois substituants choisis dans le groupe consistant en un atome d'halogène, des substituants hydroxy, alkoxy en C₁ à C₄, thiol, alkylthio en C₁ à C₄, amino, N-(alkyle en C₁ à C₄)-amino, N,N-di(alkyle en C₁ à C₄)amino, sulfonyle, alkylsulfonyle en C₁ à C₄, sulfinyle et alkylsulfinyle en C₁ à C₄ ; alcényle en C₂ à C₇ facultativement substitué avec un, deux, trois ou plus de trois atomes d'halogène ; alcynyle en C₂ à C₇ ; un groupe aryle monocyclique ou bicyclique ayant 6 à 10 atomes de carbone et des doubles liaisons alternées, ledit groupe pouvant être facultativement substitué avec un ou deux substituants choisis dans le groupe consistant en des substituants fluoro, chloro, alkyle en C₁ à C₄, cyano, nitro, hydroxy, alkoxy en C₁ à C₄, thiol, alkylthio en C₁ à C₄, amino, *N*-(alkyle en C₁ à C₄)-amino, *N,N*-di(alkyle en C₁ à C₄)amino, sulfonyle, alkylsulfonyle en C₁ à C₄, sulfinyle, alkylsulfinyle en C₁ à C₄ et pouvant être lié au reste de la molécule par n'importe quel atome de carbone disponible par une liaison directe ou par l'intermédiaire d'un groupe alkylène en C₁ à C₄ ; un groupe hétéroaryle monocyclique ou bicyclique correspondant à des groupes aromatiques et partiellement aromatiques d'un noyau monocyclique ou bicyclique ayant 4 à 12 atomes de carbone, dont au moins l'un des atomes est un hétéroatome choisi dans le groupe consistant en O, S et N, où l'atome de carbone ou d'azote disponible représente le site de liaison du groupe au reste de la molécule par une liaison directe ou par l'intermédiaire d'un groupe alkylène en C₁ à C₄ , et où ledit groupe hétéroaryle peut être facultativement substitué avec des substituants fluoro, chloro, alkyle en C₁ à C₄, cyano, nitro, hydroxy, alkoxy en C₁ à C₄, thiol, alkylthio en C₁ à C₄, amino, *N*-(alkyle en C₁ à C₄)amino, *N,N*-di(alkyle en C₁ à C₄)amino, sulfonyle, alkylsulfonyle en C₁ à C₄, sulfinyle, alkylsulfinyle en C₁ à C₄ ; un groupe hétérocyclique pentagonal ou hexagonal, totalement saturé ou partiellement insaturé, contenant au moins un hétéroatome choisi dans le groupe consistant en O, S et N, dans lequel l'atome de carbone ou d'azote disponible représente le site de liaison du groupe au reste de la molécule par une liaison directe ou par l'intermédiaire d'un groupe alkylène en C₁ à C₄, et où ledit hétérocycle peut être facultativement substitué avec des substituants fluoro, chloro, alkyle en C₁ à C₄, cyano, nitro, hydroxy, alkoxy en C₁ à C₄, thiol, alkylthio en C₁ à C₄, amino, *N*-(alkyle en C₁ à C₄)amino, *N,N*-di(alkyle en C₁ à C₄)amino, sulfonyle, alkylsulfonyle en C₁ à C₄, sulfinyle, alkylsulfinyle en C₁ à C₄ ; un groupe hydroxy ; hydroxyalcényle en C₂ à C₇ ; hydroxyalcynyle en C₂ à C₇ ; alkoxy en C₁ à C₇ ; thiol ; thioalcényle en C₂ à C₇ ; thioalcynyle en C₂ à C₇ ; alkylthio en C₁ à C₇ ; amino ; *N*-(alkyle en C₁ à C₇)amino ; *N,N-*di(alkyle en C₁ à C₇)amino ; alkylamino en C₁ à C₇ ; aminoalcényle en C₂ à C₇ ; aminoalcynyle en C₂ à C₇ ; aminoalkoxy en C₁ à C₇ ; alcanoyle en C₁ à C₇ ; aroyle en C₇ à C₁₀ ; oxoalkyle en C₁ à C₇ ; alcanoyloxy en C₁ à C₇ ; carboxy ; (alkyle en C₁ à C₇)oxycarbonyle, qui est facultativement substitué avec un, deux, trois ou plus de trois substituants choisis dans le groupe consistant en des substituants halogéno, hydroxy, alkoxy en C₁ à C₄, thiol, alkylthio en C₁ à C₄, amino, N-(alkyle en C₁ à C₄)amino, N,N-di(alkyle en C₁ à C₄)-amino, sulfonyle, alkylsulfonyle en C₁ à C₄, sulfinyle et alkylsulfinyle en C₁ à C₄ ; (aryle en C₇ à C₁₀)-oxycarbonyle, qui est facultativement substitué avec un ou deux substituants choisis dans le groupe consistant en des substituants halogéno, alkyle en C₁ à C₄, cyano, nitro, hydroxy, alkoxy en C₁ à C₄, thiol, alkylthio en C₁ à C₄, amino, N-(alkyle en C₁ à C₄)-amino, N,N-di(alkyle en C₁ à C₄)amino, sulfonyle, alkylsulfonyle en C₁ à C₄, sulfinyle, alkylsulfinyle en C₁ à C₄ ; *N*-(alkyle en C₁ à C₇)carbamoyle ; *N,N-*di(alkyle en C₁ à C₇)carbamoyle ; cyano ; cyano(alkyle en C₁ à C₇) ; sulfonyle ; alkylsulfonyle en C₁ à C₇ ; sulfinyle ; alkylsulfinyle en C₁ à C₇ ; nitro ;
ou un substituant représenté par la formule II : 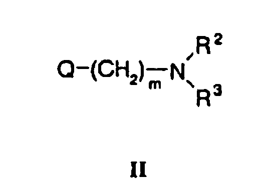 dans laquelle
R² et R³, simultanément ou indépendamment l'un de l'autre, représentent un atome d'hydrogène, un groupe alkyle en C₁ à C₄, ou un groupe aryle répondant à la définition précitée ou bien, conjointement avec N, représentent un hétérocycle ou un groupe hétéroaryle facultativement substitué, où le terme "hétérocycle" désigne un groupe hétérocyclique pentagonal ou hexagonal totalement saturé ou partiellement insaturé contenant au moins un hétéroatome choisi dans le groupe consistant en O, S et N, et où ledit hétérocycle pouvant être facultativement substitué avec un ou deux substituants qui sont choisis entre des substituants halogéno, alkyle en C₁ à C₄, cyano, nitro, hydroxy, alkoxy en C₁ à C₄, thiol, alkylthio en C₁ à C₄, amino, *N*-(alkyle en C₁ à C₄)amino, *N,N-*di(alkyle en C₁ à C₄)amino, sulfonyle, alkylsulfonyle en C₁ à C₄, sulfinyle, alkylsulfinyle en C₁ à C₄ ; où le terme "hétéroaryle" désigne des groupes aromatiques et partiellement aromatiques d'un noyau monocyclique ou bicyclique ayant 4 à 12 atomes de carbone dont au moins un des atomes est un hétéroatome choisi dans le groupe consistant en O, S et N et où ledit groupe hétéroaryle peut être facultativement substitué avec un ou deux substituants qui sont choisis entre des substituants halogéno, alkyle en C₁ à C₄, cyano, nitro, hydroxy, alkoxy en C₁ à C₄, thiol, alkylthio en C₁ à C₄, amino, *N*-(alkyle en C₁ à C₄)amino, *N,N-*di(alkyle en C₁ à C₄)amino, sulfonyle, alkylsulfonyle en C₁ à C₄, sulfinyle et alkylsulfinyle en C₁ à C₄ ;
m représente un nombre entier de 1 à 3 ; et
Q représente un atome d'oxygène, un atome de soufre ou un groupe NH ;
et ses sels et produits de solvatation pharmacologiquement acceptables,
qui comprend :
a) la condensation d'un composé Ia : 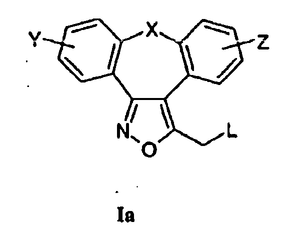 dans lequel les symboles X, Y et Z répondent aux définitions précitées, L représente un groupe partant,
avec un alcool, un thioalcool ou une amine choisi facultativement, ou avec un composé de formule IIa : 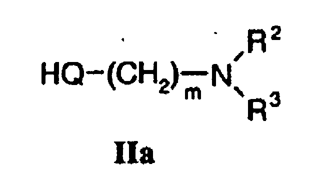 où tous les radicaux et symboles répondent aux définitions précitées ; ou
b) la condensation d'un composé de formule Ib : 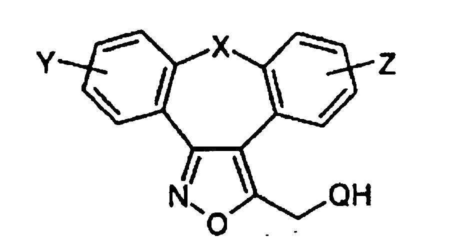
dans laquelle tous les symboles répondent aux définitions précitées, avec un composé de formule IIb : 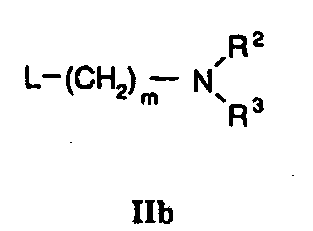 dans laquelle les radicaux R² et R³ et le symbole m répondent aux définitions précitées et le symbole L représente un groupe partant convenable.

10. Composition pharmaceutique comprenant au moins un composé suivant la revendication 1 ou un de ses sels ou produits de solvatation pharmaceutiquement acceptables en association avec un excipient, diluant et/ou support pharmaceutiquement acceptable.

11. Utilisation d'un composé suivant la revendication 1, pour la production d'une formulation pharmaceutique destinée au traitement et à la prévention de maladies, d'altérations et de troubles du système nerveux central provoqués par des troubles de l'équilibre neurochimique d'amines biogènes ou d'autres neurotransmetteurs.

12. Utilisation suivant la revendication 11, dans laquelle les amines biogènes choisies sont la sérotonine, la norépinéphrine et la dopamine.

13. Utilisation suivant la revendication 11, dans laquelle le neurotransmetteur est le glutamate.

14. Utilisation suivant la revendication 11, 12 ou 13, dans laquelle les composés de formule générale I agissent sur l'équilibre neurochimique en régulant la synthèse, le stockage, la libération, le métabolisme et/ou la réabsorption d'amines biogènes ou de neurotransmetteurs et la liaison à leurs récepteurs.

15. Utilisation suivant la revendication 14, dans laquelle les composés de formule générale I présentent une affinité de liaison pour un récepteur d'une ou plusieurs amines biogènes.

16. Utilisation suivant la revendication 15, dans laquelle les composés de formule générale I présentent une affinité de liaison significative pour les récepteurs de sérotonine 5-HT_{2A} et 5-HT_{2C}.

17. Utilisation suivant la revendication 16, dans laquelle les composés de formule générale I présentent une affinité de liaison pour des récepteurs de sérotonine choisis à une concentration CI₅₀ < 1µM.

18. Utilisation suivant la revendication 11, dans laquelle les composés de formule générale I jouent le rôle de ligands du récepteur σ1 à une concentration CI₅₀ < 1µM en modulant le système de neurotransmetteurs central.

19. Utilisation suivant la revendication 11, 16 ou 18, dans laquelle les composés de formule générale I présentent une double affinité de liaison pour le récepteur σ1 et au moins un récepteur de sérotonine choisi entre les récepteurs 5-HT_{2A} et 5-HT_{2C}.

20. Utilisation suivant la revendication 11, dans laquelle les maladies et troubles du système nerveux central sont choisis dans le groupe consistant en l'anxiété, la dépression et la dépression modérée, des troubles bipolaires, des troubles du sommeil, des troubles sexuels, la psychose, la psychose à la limite de la normale, la schizophrénie, la migraine, des troubles de la personnalité, des troubles obsessionnels compulsifs, la phobie sociale ou les attaques de panique, des troubles mentaux organiques chez les enfants, l'agression, des troubles de la mémoire et des troubles de la personnalité chez les sujets âgés, la toxicomanie, l'obésité, la boulimie et des troubles similaires, les ronflements et les troubles prémenstruels.

21. Utilisation suivant la revendication 11, dans laquelle les altérations du système nerveux central sont provoquées par un traumatisme, un ictus cérébral, des maladies neurodégénératives, des troubles cardio-vasculaires tels qu'une élévation de la pression sanguine, une thrombose, un infarctus et également par des troubles gastro-intestinaux.

22. Utilisation suivant la revendication 11, dans laquelle les composés de formule générale I et leurs sels et produits de solvatation pharmaceutiquement acceptables sont choisis dans le groupe consistant en :
le 3-méthyl-2-oxa-8-thia-1-aza-dibenzo[*e,h*]azulène ;
le 11-chloro-3-méthyl-2-oxa-8-thia-1-aza-dibenzo[*e,h*]-azulène ;
le 3-méthyl-2,8-dioxa-1-aza-dibenzo[*e,h*]azulène ;
le 3-bromométhyl-2-oxa-8-thia-1-aza-dibenzo[*e,h*]azulène ;
le 3-bromométhyl-11-chloro-2-oxa-8-thia-1-aza-dibenzo-[*e,h*]azulène ;
le 3-bromométhyl-2,8-dioxa-1-aza-dibenzo[*e,h*]azulène ;
la diméthyl-[2-(2-oxa-8-thia-1-aza-dibenzo[*e,h*]azulène-3-ylméthoxy)-éthyl]-amine ;
la diméthyl-[3-(2-oxa-8-thia-1-aza-dibenzo[*e,h*]azulène-3-ylméthoxy)-propyl]-amine ;
la diméthyl-[2-(11-chloro-2-oxa-8-thia-1-aza-dibenzo-[*e,h*]azulène-3-ylméthoxy)-éthyl]-amine ;
la diméthyl-[3-(11-chloro-2-oxa-8-thia-1-aza-dibenzo-[*e,h*]azulène-3-ylméthoxy)-propyl]-amine ;
la diméthyl-[2-(2,8-dioxa-1-aza-dibenzo[*e,h*]azulène-3-ylméthoxy)-éthyl]-amine ; et
la diméthyl-[3-(2,8-dioxa-1-aza-dibenzo[*e,h*]azulène-3-ylméthoxy)-propyl]-amine.
